# EUROPEAN PATENT SPECIFICATION

(11) **EP 3 311 762 B1**
(45) Date of publication and mention of the grant of the patent: **22.07.2020**
(21) Application number: 17199103.7
(22) Date of filing: 19.06.2006
(51) Int. Cl.: A61B 17/20, A61K 9/00, A61K 9/70

(54) **PERMEANT DELIVERY SYSTEM AND METHODS FOR USE THEREOF**
DURCHDRINGENDES ABGABESYSTEM UND ANWENDUNGSVERFAHREN DAFÜR
SYSTEME DE DELIVRANCE DE PERMEANT ET PROCEDES D'UTILISATION

(30) Priority: 17.06.2005 US 691898 P
(43) Date of publication of application: 25.04.2018
(62) Divisional of application: 06773439.2
(73) Proprietor: PassPort Technologies, Inc., San Diego, CA 92121 (US)
(72) Inventor: Tolia, Gaurav, Norcross, GA 30093 (US); Tagliaferri, Frank, Decatur, GA 30030 (US); Enscore, David, Alpharetta, GA 30022 (US); Eppstein, Jonathan, Atlanta, GA 30319 (US); Smith, Alan, Atlanta, GA 30305-2733 (US); McRae, Stuart, Decatur, GA 30033 (US); Chang, Shulun, Atlanta, GA 30308 (US); Patel, Yogi, Lawrenceville, GA 30044 (US)
(74) Representative: Hoefer & Partner Patentanwälte mbB

(56) References cited:
- WO-A1-02/074286
- WO-A2-00/74767
- WO-A2-97/10812
- WO-A2-2004/039428
- WO-A2-2006/015299
- FR-A1- 2 562 800
- US-A- 5 032 109
- US-A1- 2004 087 893

## Description

### Field of the Invention

The present invention relates generally to the field of transdermal permeant delivery and more specifically to devices, systems and methods for same.

### Background of the Invention

Transdermal drug delivery systems have been marketed for a variety of therapeutic indications over the past 20 years. Typically, transdermal delivery systems are fabricated as multilayered polymeric laminates in which a drug reservoir or a drug-polymer matrix is sandwiched between two polymeric layers: an outer impervious backing layer that creates an occlusive environment and prevents the loss of drug through the backing surface and an inner polymeric layer that functions as an adhesive and/or rate-controlling membrane. In the case of a drug reservoir design, the reservoir is sandwiched between the backing and a rate controlling membrane. The drug releases only through the rate-controlling membrane, which can be microporous or nonporous. In the drug reservoir compartment, the drug can be in the form of a solution, suspension, or gel or dispersed in a solid polymer matrix. On the outer surface of the polymeric membrane a thin layer of drug-compatible, hypoallergenic adhesive polymer may be applied.

In the case of the drug matrix design, there are two types, the drug-in-adhesive system and the matrix dispersion system. In the drug-in-adhesive system, the drug reservoir is formed by dispersing the drug in an adhesive polymer and then spreading the medicated polymer adhesive by solvent casting or by melting the adhesive (in the case of hot-melt adhesives) onto an impervious backing layer. On top of the reservoir, layers of unmedicated adhesive polymer are applied. In the case of the matrix dispersion system, the drug is dispersed homogeneously in a hydrophilic or lipophilic polymer matrix and fixed onto a drug-impermeable backing layer. Instead of applying the adhesive on the face of the drug reservoir, it is applied to form a peripheral adhesive.

Most conventional transdermal products contain small molecule drugs (< 500 Daltons) that are lipophilic in nature, allowing them to dissolve into and diffuse through the lipid bilayers of the outer layer of the skin, the stratum corneum. Most transdermal products contain the lipophilic base form of the drug, not the hydrophilic or water soluble salt form. Transdermal delivery is typically limited to small molecules to allow a sufficient flux into the body across a reasonably sized patch area. To increase transdermal flux, chemical permeation enhancers have been added to transdermal formulations. However, use of chemical permeation enhancers has not been successful achieving a sufficient flux of a hydrophilic or water soluble drug or any molecule larger than 1000 Daltons to reach therapeutic levels. Accordingly, there is a need in the art for improved methods, systems and devices for achieving transdermal delivery of a hydrophilic permeant to a subject at therapeutic delivery rates.
Patent document WO 2004/039428 discloses transdermal delivery systems for dried particulate or lyophilized medications.

### Summary of the Invention

The present invention provides devices, systems and methods for causing the transdermal flux of a permeant through at least one formed pathway through a skin layer of a subject.
To this end, in a first aspect, the present invention provides a device for causing the transdermal flux of a permeant into a subject via at least one formed pathway through a skin layer of the subject, comprising an anhydrous delivery reservoir comprising a non-biodegradable matrix comprising a water-insoluble polymer, having a bottom surface and defining a plurality of conduits therein the matrix, at least a portion of the plurality of conduits being in communication with the bottom surface; and an undissolved hydrophilic permeant disposed therein at least a portion of the plurality of conduits of the matrix, the hydrophilic permeant comprising at least one bioactive agent and at least one biocompatible filler, wherein the hydrophilic permeant can come in contact with subcutaneous fluid from the subject when the bottom surface of the matrix is positioned in fluid communication with the at least one formed pathway.
In a second aspect, the present invention provides a system for causing the transdermal flux of a permeant into a subject via at least one formed pathway through a skin layer of the subject. According to this aspect of the invention, the system comprises a means for intentionally forming the at least one pathway in the skin layer and at least one anhydrous delivery reservoir according to the present invention as described herein.

In a third aspect, the present description discloses a method for causing the transdermal flux of a permeant into a subject via at least one formed pathway through a skin layer of the subject. According to this aspect, the method comprises providing a subject having a transdermal permeant administration site, the administration site comprising the at least one formed pathway through the skin layer and also providing a delivery reservoir according to the instant invention as described herein. The delivery reservoir is placed in fluid communication with the at least one formed pathway through the skin layer and maintained in fluid communication with the at least one formed pathway through the skin layer to draw subcutaneous fluid from the subject from the at least one formed pathway and to subsequently transdermally deliver permeant at a desired flux through the at least one formed pathway.

Additional aspects of the invention will be set forth, in part, in the detailed description, figures and any claims which follow, and in part will be derived from the detailed description, or can be learned by practice of the invention. It is to be understood that both the foregoing general description and the following detailed description are exemplary and explanatory only and are not restrictive of the invention as disclosed.

### Brief Description of the Figures

The accompanying drawings, which are incorporated in and constitute a part of this specification, illustrate certain aspects of the instant invention and together with the description, serve to explain, without limitation, the principles of the invention.
Figure 1 illustrates a side view of a permeant delivery reservoir according to one aspect of the present invention.
Figure 2 illustrates a side view of a permeant delivery reservoir according to one aspect of the present invention wherein the delivery reservoir comprises an enhanced surface area provided by perforations.
Figure 3 illustrates a side view of a permeant delivery reservoir according to one aspect of the present invention wherein the reservoir comprises a plurality of delivery reservoirs positioned in a stacked arrangement.
Figure 4 illustrates an exemplary transdermal permeant delivery patch according to one aspect of the present invention.
Figure 5 illustrates a schematic diagram of an electro-osmotic pump assembly according to one aspect of the present invention.
Figure 6 illustrates an exemplary transdermal permeant delivery patch according to one aspect of the present invention wherein the patch assembly further comprises a first, second and third electrode assembly.
Figure 7 is a chart reporting exemplary in vitro release kinetics for a permeant delivery reservoir of the present invention.
Figure 8 is a chart reporting exemplary pharmacokinetic profile data for a permeant delivery reservoir according to one aspect of the present invention.
Figure 9 is a chart reporting the effect of reservoir thickness on exemplary pharmacokinetic profiles provided by permeant reservoirs according to one aspect of the present invention.
Figure 10 is a chart reporting a comparison of exemplary drug utilization achieved by an aqueous delivery reservoir compared to a delivery reservoir according to one aspect of the present invention.
Figure 11 is a chart reporting the effect of drug reservoir thickness on utilization for exemplary delivery reservoirs according to one aspect of the present invention.
Figure 12 is a chart reporting the mean pharmacokinetic profile (PK profile) for an exemplary permeant delivery reservoir device according to one aspect of the present invention.
Figure 13 is a chart reporting data illustrating the exemplary ability to optimize the drug utilization of a given permeant delivery reservoir according to one aspect of the present invention.
Figure 14 is a chart reporting the effect of pore density within a permeant administration site on the mean pharmacokinetic profile of a permeant reservoir according to one aspect of the present invention.
Figure 15 is a chart reporting the effect of pore density on mean hydromorphone serum concentration during a 6-24 hour administration period using a permeant delivery reservoir according to one aspect of the present invention.
Figure 16 is a chart reporting mean serum hydromorphone concentration data from test subjects resulting from the administration of an exemplary permeant delivery reservoir according to one aspect of the present invention.
Figure 17 is a chart reporting a comparison of an exemplary pharmacokinetic profile resulting from an aqueous delivery reservoir compared to a delivery reservoir according to one aspect of the present invention.
Figure 18 is a chart reporting mean cumulative insulin release kinetics for a permeant delivery reservoir according to one aspect of the present invention.
Figure 19 is a chart reporting the mean serum insulin concentration levels among subjects that were administered insulin via a delivery reservoir according to one aspect of the present invention.
Figure 20 is a chart reporting mean changes in serum glucose concentrations among subjects that were administered insulin transdermally via a permeant delivery reservoir according to one aspect of the present invention.
Figure 21 is a chart reporting a comparison of serum hydromorphone PK profiles among test subjects that were administered hydromorphone transdermally via a permeant delivery reservoir of the present invention comprising propylene glycol and among test subjects that were administered hydromorphone transdermally via a permeant delivery reservoir without propylene glycol.
Figure 22 reports data from an *in vitro* dissolution study comparing the percentage of hydromorphone released from a hydromorphone file without glycerin compared to a hydromorphone film also comprising 1.0 weight percent glycerin.
Figure 23 reports data from an *in vivo* hairless rat pharmacokinetic study showing the effect of increasing the glycerin percentage on steady state hydromorphone serum levels.
Figure 24 reports serum hydromorphone PK profiles from a Phase 1 clinical study showing the effect of adding 1.0 weight % glycerin to a hydromorphone polymer film of the present invention.
Figure 25 reports percentage of fentanyl citrate released as a function of time at 32° C for a permeant delivery reservoir according to one aspect of the present invention.
Figure 26 reports the effects of changes in the polymer and fentanyl citrate loading on serum drug concentrations in the hairless rat for permeant delivery reservoirs according to the present invention.
Figure 27 reports mean insulin serum level PK profiles for permeant delivery reservoirs according to another aspect of the present invention.
Figure 28 reports the enhancing effect glycerin can have on the mean insulin serum level PK profiles for permeant delivery reservoirs according to another aspect of the present invention.

### Detailed Description of the Invention

The present invention can be understood more readily by reference to the following detailed description, examples, and claims, and their previous and following description.

Before the present compositions, devices, and/or methods are disclosed and described, it is to be understood that this invention is not limited to the specific articles, devices, and/or methods disclosed unless otherwise specified. It is also to be understood that the terminology used herein is for the purpose of describing particular aspects only and is not intended to be limiting.

The following description of the invention is provided as an enabling teaching of the invention in its best, currently known embodiment. Those skilled in the relevant art will recognize that many changes can be made to the embodiments described, while still obtaining the beneficial results of the present invention. It will also be apparent that some of the desired benefits of the present invention can be obtained by selecting some of the features of the present invention without utilizing other features. Accordingly, those who work in the art will recognize that many modifications and adaptations to the present invention are possible and can even be desirable in certain circumstances and are a part of the present invention. Thus, the following description is provided as illustrative of the principles of the present invention and not in limitation thereof.

As used herein, the singular forms "a," "an" and "the" include plural referents unless the context clearly dictates otherwise. Thus, for example, reference to a "permeant delivery reservoir" includes aspects having two or more permeant delivery reservoirs unless the context clearly indicates otherwise.

Ranges can be expressed herein as from "about" one particular value, and/or to "about" another particular value. When such a range is expressed, another aspect includes from the one particular value and/or to the other particular value. Similarly, when values are expressed as approximations, by use of the antecedent "about," it will be understood that the particular value forms another aspect. It will be further understood that the endpoints of each of the ranges are significant both in relation to the other endpoint, and independently of the other endpoint.

As used herein, the terms "optional" or "optionally" mean that the subsequently described event or circumstance may or may not occur, and that the description includes instances where said event or circumstance occurs and instances where it does not.

As used herein, a "weight percent" or "percent by weight" of a component, unless specifically stated to the contrary, is based on the total weight of the formulation or composition in which the component is included.

As used herein, the term or phrase "effective," "effective amount," or "conditions effective to" refers to such amount or condition that is capable of performing the function or property for which an effective amount is expressed. As will be pointed out below, the exact amount or particular condition required will vary from one embodiment to another, depending on recognized variables such as the materials employed and the processing conditions observed. Thus, it is not always possible to specify an exact "effective amount" or "condition effective to." However, it should be understood that an appropriate effective amount or effective condition will be readily determined by one of ordinary skill in the art using only routine experimentation.

As used herein, the term "hydrophilic permeant" refers in one aspect to a permeant having an affinity for subcutaneous fluid. In one aspect, the subcutaneous fluid can be intracellular and/or extracellular fluid. In one aspect, a hydrophilic permeant can be at least substantially water-soluble such that once contacted with a water or moisture source, such as subcutaneous fluid, the hydrophilic permeant at least substantially dissolves in the subcutaneous fluid. In another aspect, the hydrophilic permeant may not substantially dissolve in the subcutaneous fluid but rather may form a suspension of the microparticulate hydrophilic permeant in the subcutaneous fluid.

As used herein, a "subcutaneous fluid" can include, without limitation, moisture, plasma, blood, one or more proteins, interstitial fluid, skin tissue fluid, perspiration, serum, lymphatic fluid, and/or any combination of two or more thereof. In one aspect, a subcutaneous fluid according to the instant invention is a moisture source comprising water.

As used herein, the term "non-biodegradable" refers to a material, compound or composition, which at least substantially does not degrade or erode when contacted by subcutaneous fluid. In one aspect, a non-biodegradable material, compound or composition can be a substantially water-insoluble material, compound, or composition.

As used herein, the term "permeant utilization" refers to the percentage of the initial permeant content disposed within a permeant delivery reservoir that is transdermally delivered from reservoir to a subject during a predetermined permeant administration period.

As used herein, a "subject" refers to any living organism having at least one outer membrane through which fluid can be obtained. In one aspect, an exemplary outer membrane can be at least one skin layer through which subcutaneous fluid can be obtained. For example, in one aspect a subject can be a plant. Alternatively, in another aspect, the subject can be an animal. In one aspect the animal can be mammalian. In an alternative aspect the animal can be non-mammalian. The animal can also be a cold-blooded animal, such as a fish, a reptile, or an amphibian. Alternatively, the animal can be a warm-blooded animal, such as a human, a farm animal, a domestic animal, or even a laboratory animal. Accordingly, it should be understood that the present invention is not limited to its use in connection with any one particular subject or group of subjects.

As used herein, a "skin layer" can be any one or more epidermal layers of a subject. For example, in one aspect, a skin layer includes the outermost layer of the skin, *i.e.,* the stratum corneum. In an alternative aspect, a skin layer can include one or more backing layers of the epidermis, commonly identified as stratum granulosum, stratum malpighii, and stratum germinativum layers. It will be appreciated by one of ordinary skill in the art that there is essentially little or no resistance to transport or to absorption of a permeant through the backing layers of the epidermis. Therefore, in one aspect of the present invention, an at least one formed pathway in a skin layer of a subject is a pathway in the stratum corneum layer of a subject.

As used herein, "enhancer," "chemical enhancer," "penetration enhancer," "permeation enhancer," and the like includes all enhancers that increase the flux of a permeant, analyte, or other molecule across the biological membrane, and is limited only by functionality. In other words, all cell envelope disordering compounds and solvents and any other chemical enhancement agents are intended to be included. Additionally, all active force enhancer technologies such as the application of sonic energy, mechanical suction, pressure, or local deformation of the tissues, sonophoresis, iontophoresis or electroporation are included. One or more enhancer technologies may be combined sequentially or simultaneously. For example, a chemical enhancer may first be applied to permealize the capillary wall and then an iontophoretic or sonic energy field may be applied to actively drive a permeant into those tissues surrounding and comprising the capillary bed.

As used herein, "transdermal" or "percutaneous" includes the passage of a permeant into and through the biological membrane to achieve effective therapeutic blood levels or local tissue levels of a permeant.

As used herein, the term "biological membrane" or "tissue membrane" means the structure separating one area of an organism from another, such as a capillary wall, lining of the gut or the outer layer of an organism which separates the organism from its external environment, such as epithelial tissue, skin, buccal mucosa or other mucous membrane. The stratum corneum of the skin may also be included as a biological membrane.

As used herein, "artificial opening" or "micropore" means any physical breach of the biological membrane of a suitable size for delivering or extraction fluid therethrough, including micropores. "Artificial opening" or "micropore" or any such similar term thus refers to a small hole, opening or crevice created to a desired depth in or through a biological membrane. The opening could be formed via the conduction of thermal energy as described in U.S. Pat. No. 5,885,211, or through a mechanical process, or through a pyrotechnic process. The size of the hole or pore is for example approximately 1-1000 microns in diameter. It is to be understood that the term micropore is used in the singular form for simplicity, but that the devices and methods may form multiple openings or pores.

As used herein, "iontophoresis" refers to the application of an external electric field to the tissue surface through the use of two or more electrodes and delivery of an ionized form of drug or an un-ionized drug carried with the water flux associated with ion transport (electro-osmosis) into the tissue or the similar extraction of a biological fluid or analyte.

As used herein, "electroporation" refers to the creation through electric current flow of openings in cell walls that are orders of magnitude smaller than micropores. The openings formed with electroporation are typically only a few nanometers in any dimension. In one example, electroporation is useful to facilitate cellular uptake of selected permeants by the targeted tissues beneath the outer layers of an organism after the permeant has passed through the micropores into these deeper layers of tissue.

As used herein, "sonophoresis" or "sonification" refers to sonic energy, which may include frequencies normally described as ultrasonic, generated by vibrating a piezoelectric crystal or other electromechanical element by passing an alternating current through the material. The use of sonic energy to increase the permeability of the skin to drug molecules has been termed sonophoresis or phonophoresis.

The present invention is based, in part, upon new approaches to transdermal delivery that have been developed through increasing a subjects skin permeability by physically altering it via the formation of tiny, artificial openings through at least one layer of the skin. These openings can provide fluid access into the hydrated, living layers of the epidermal and dermal skin tissues beneath the stratum corneum layer. To that end, these openings, or micropores, can be viewed as aqueous channels, through which not only permeant can diffuse, but fluid can be pumped, micro-particles can be delivered, or fluid from within the subject can exude to the surface of the skin. By utilizing the bi-directional properties of fluid flow and micropores of this type the present invention provides, in one aspect, improved devices, systems and methods of transdermal permeant delivery as described in detail below.

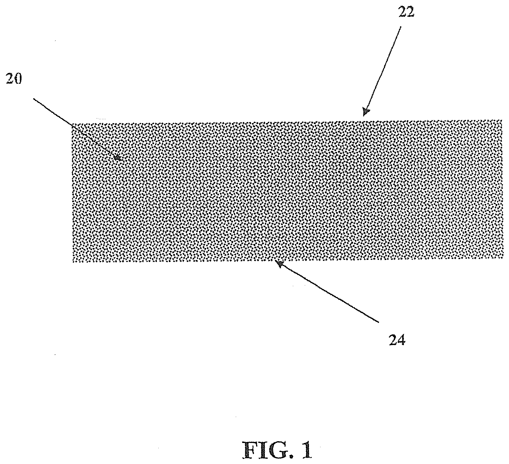

As briefly summarized above and as illustrated in Figure 1, in a first aspect the present invention provides a device **10** for causing the transdermal flux of a permeant into a subject via at least one formed pathway through a skin layer of the subject. The device is comprised of a permeant delivery reservoir as defined in the claims **20** having a top surface **22** and an opposed bottom surface **24** and comprising at least one undissolved hydrophilic permeant disposed therein. The hydrophilic permeant can come in contact with subcutaneous fluid when the bottom surface of the reservoir is position in fluid communication with the at least one formed pathway through the skin layer of a subject. Once an effective amount of subcutaneous fluid has come into contact with the delivery reservoir, the fluid subsequently provides a diffusion path for transdermally delivering at least a portion of the permeant back through the skin into the subject. For example, in one aspect and without limitation, the hydrophilic permeant can have an affinity for subcutaneous fluid such that at least a portion of the undissolved permeant can draw an effective amount of subcutaneous fluid from the subject when the bottom surface of the reservoir is positioned in fluid communication with the at least one formed pathway through the skin layer of a subject.

It will be appreciated upon practicing the present invention that in one aspect an undissolved hydrophilic permeant disposed within the non-biodegradable matrix is not transdermally active or available for transdermal delivery until first coming in contact with subcutaneous fluid drawn from the subject.

Furthermore, conventional implantable or oral delivery systems using highly water-soluble drug forms typically experience a burst effect seen in the resulting PK profiles. However, by keeping the reservoir of hydrophilic permeant on the skin surface, and providing a reservoir that can ensure a specified release rate, this burst effect can be eliminated by the delivery reservoirs of the instant invention.

The permeant delivery reservoir comprises a non-biodegradable matrix which, as stated above, further comprises at least one hydrophilic permeant disposed therein. The matrix component of the permeant delivery reservoir is comprised of a non-biodegradable material or combination of non-biodegradable materials that are biocompatible for topical application to the outer skin layer of a subject for extended permeant application periods. The non-biodegradable material can, in one non-limiting aspect, account for approximately 20 weight % to approximately 80 weight % of the permeant delivery reservoir, including additional amounts as 25 weight %, 30 weight %, 35 weight %, 40 weight %, 45 weight %, 50 weight %, 55 weight %, 60 weight %, 65 weight %, 70 weight %, and 75 weigh% of the permeant delivery reservoir, and including any range of weight percentages derived from these values.

In one aspect, the non-biodegradable matrix can comprise a non-biodegradable polymeric material or combination of polymeric materials. In one aspect, the non-biodegradable polymeric material is water-insoluble and, optionally, also hydrophobic. For example and without limitation, in one aspect, the non-biodegradable matrix can comprise an ethylene vinyl acetate (EVA) co-polymer; polyethylene, polyethyl acrylate, and copolymers of ethylene and ethyl acrylate, and any combination thereof. In one aspect, the matrix is comprised of an ethylene-vinyl acetate co-polymer having a relative percentage of vinyl acetate in the range of from 0% to approximately 60%, including additional vinyl acetate percentages as approximately 0%, 1%, 5%, 10%, 15%, 20%, 25%, 30%, 35%, 40%, 45 %, 50%, 55% and 60% and any range of percentages derived from these values. In still another aspect, the ethylene-vinyl acetate co-polymer comprises approximately 28% vinyl acetate.

The hydrophilic permeant can comprise any chemical or biological material, compound, or composition suitable for administration by the conventional methods previously known in the art and/or by the methods taught in the present invention. To this end, the permeant can comprise any one or more components that would be desired to be administered transdermally. For example, the hydrophilic permeant can be selected from a bioactive agent, a filler, an anti-healing agent, an osmotic agent, and any other conventionally known additive suitable for providing or enhancing a desired transdermal delivery of a permeant. In one aspect, the hydrophilic permeant can account for approximately 20 weight % to approximately 80 weight % of the permeant delivery reservoir, including additional amounts as 25 weight %, 30 weight %, 35 weight %, 40 weight %, 45 weight %, 50 weight %, 55 weight %, 60 weight %, 65 weight %, 70 weight %, and 75 weight % of the permeant delivery reservoir, and including any range of weight percentages derived from these values.

As used herein, a "bioactive agent" includes any drug, chemical, or biological material that induces a desired biological or pharmacological effect. The effect can be local, such as providing for a local anesthetic effect, or it can be systemic. Such substances include broad classes of compounds normally delivered into the body, including through body surfaces and membranes, including skin. To this end, in one aspect, the bioactive agent can be a small molecule agent. In another aspect, the bioactive agent can be a macromolecular agent. In general, and without limitation, exemplary bioactive agents include, but are not limited to, anti-infectives such as antibiotics and antiviral agents; analgesics and analgesic combinations; anorexics; antihelminthics; antiarthritics; antiasthmatic agents; anticonvulsants; antidepressants; antidiabetic agents; antidiarrheals; antihistamines; antiinflammatory agents; antimigraine preparations; antinauseants; antineoplastics; antiparkinsonism drugs; antipruritics; antipsychotics; antipyretics; antispasmodics; anticholinergics; sympathomimetics; xanthine derivatives; cardiovascular preparations including potassium and calcium channel blockers, beta-blockers, alpha-blockers, and antiarrhythmics; antihypertensives; diuretics and antidiuretics; vasodilators including general coronary, peripheral, and cerebral; central nervous system stimulants; vasoconstrictors; cough and cold preparations, including decongestants; hormones such as estradiol and other steroids, including corticosteroids; hypnotics; immunosuppressives; muscle relaxants; parasympatholytics; psychostimulants; sedatives; and tranquilizers

The devices and methods of the instant invention can also be used to transdermally delivery peptides, polypeptides, proteins, or other macromolecules known to be difficult to deliver transdermally with existing conventional techniques because of their size. These macromolecular substances typically have a molecular weight of at least about 300 Daltons, and more typically, in the range of about 300 to 40,000 Daltons. Examples of polypeptides and proteins which may be delivered in accordance with the present invention include, without limitation, antibodies, LHRH, LHRH analogs (such as goserelin, leuprolide, buserelin, triptorelin, gonadorelin, napharelin and leuprolide), GHRH, GHRF, insulin, insulinotropin, calcitonin, octreotide, endorphin, TRH, NT-36 (chemical name: N-[[(s)-4-oxo-2-azetidinyl]-carbonyl]-L-histidyl-L-prolinamide), liprecin, pituitary hormones (eg, HGH, HMG, HCG, desmopressin acetate, etc), follicle luteoids, .alpha.-ANF, growth factor such as releasing factor (GFRF), .beta.-MSH, GH, somatostatin, bradykinin, somatotropin, platelet-derived growth factor, asparaginase, bleomycin sulfate, chymopapain, cholecystokinin, chorionic gonadotropin, corticotropin (ACTH), erythropoietin, epoprostenol (platelet aggregation inhibitor), glucagon, hirudin and hirudin analogs such as hirulog, hyaluronidase, interleukin-2, menotropins (urofollitropin (FSH) and LH), oxytocin, streptokinase, tissue plasminogen activator, urokinase, vasopressin, desmopressin, ACTH analogs, ANP, ANP clearance inhibitors, angiotensin II antagonists, antidiuretic hormone agonists, antidiuretic hormone antagonists, bradykinin antagonists, CD4, ceredase, CSI's, enkephalins, FAB fragments, IgE peptide suppressors, IGF-1, neurotrophic factors, colony stimulating factors, parathyroid hormone and agonists, parathyroid hormone antagonists, prostaglandin antagonists, cytokines, lymphokines, pentigetide, protein C, protein S, renin inhibitors, thymosin alpha-1, thrombolytics, TNF, GCSF, EPO, PTH, heparin having a molecular weight from 3000 to 12,000 daltons, vaccines, vasopressin antagonist analogs, interferon-.alpha., -.beta., and -.gamma., alpha-1 antitrypsin (recombinant), and TOP-beta. genes; peptides; polypeptides; proteins; oligonucleotides; nucleic acids; and polysaccharides.

As used herein, "peptide", means peptides of any length and includes proteins. The terms "polypeptide" and "oligopeptide" are used herein without any particular intended size limitation, unless a particular size is otherwise stated. Exemplary peptides that can be utilized include, without limitation, oxytocin, vasopressin, adrenocorticotrophic hormone, epidermal growth factor, prolactin, luliberin or luteinising hormone releasing hormone, growth hormone, growth hormone releasing factor, insulin, somatostatin, glucagon, interferon, gastrin, tetragastrin, pentagastrin, urogastroine, secretin, calcitonin, enkephalins, endorphins, angiotensins, renin, bradykinin, bacitracins, polymixins, colistins, tyrocidin, grarnicidines, and synthetic analogues, modifications and pharmacologically active fragments thereof, monoclonal antibodies and soluble vaccines. It is contemplated that the only limitation to the peptide or protein drug which may be utilized is one of functionality.

Examples of peptide and protein drugs that contain one or more amino groups include, without limitation, anti-cancer agents, antibiotics, anti-emetic agents, antiviral agents, anti-inflammatory and analgesic agents, anesthetic agents, anti-ulceratives, agents for treating hypertension, agents for treating hypercalcemia, agents for treating hyperlipidemia, etc., each of which has at least one primary, secondary or tertiary amine group in the molecule, preferably, peptides, proteins or enzymes such as insulin, calcitonin, growth hormone, granulocyte colony-stimulating factor(G-CSF), erythropoietin (EPO), bone morphogenic protein (BMP), interferon, interleukin, platelet derived growth factor (PDGF), vascular endothelial growth factor (VEGF), fibroblast growth factor (FGF), nerve growth factor (NGF), urokinase, etc. can be mentioned. Further examples of protein drugs include, without limitation, insulin, alpha-, beta-, and gamma-interferon, human growth hormone, alpha- and beta-1-transforming growth factor, granulocyte colony stimulating factor (G-CSF), granulocyte macrophage colony stimulating factor (G-MCSF), parathyroid hormone (PTH), human or salmon calcitonin, glucagon, somatostatin, vasoactive intestinal peptide (VIP), and LHRH analogs.

In still another aspect, the bioactive agent can be present within the delivery reservoir as an undissolved anhydrous hydrophilic salt. To that end, as used herein, "hydrophilic salt" and similar terms include, without limitation, an ionic form of a bioactive agent, drug, or pharmaceutical agent, such as sodium, potassium, ammonium, trimethamin, or other cation salts thereof, sulfate or other anion salts thereof, acid addition salts of basic drugs, and base addition salts of acidic drugs. Illustrative examples of such salts include sodium diclofenac, sodium cromolyn, sodium acyclovir, sodium ampicillin, sodium warfarin, ketorolac tromethamine, amloride HCl, ephedrine HCl, loxapine HCl, thiothixene HCl, trifluoperizine HCl, naltrexone HCl, naloxone HCl, nalbuphine HCl, buspirone HCl, bupriprion HCl, phenylephrine HCl, tolazoline HCl, chlorpheniramine maleate, phenylpropanolamine HCl, clonidine HCl, dextromethorphan HBr, metoprolol succinate, metoprolol tartrate, epinephrine bitartrate, ketotofin fumarate, atropine sulfate, fentanyl citrate, apomorphine sulfate, propranolol HCl, pindolol HCl, lidocaine HCl, tetracycline HCl, oxytetracycline HCl, tetracaine HCl, dibucaine HCl, terbutaline sulfate, scopolamine HBr, brompheniramine maleate and hydromorphone HCl.

In addition to one or more bioactive agents, the permeant also comprises a bio-compatible filler. The permeant or filler can also comprise any one or more of an excipient, hygroscopic agent, osmotic agent, permeation, enhancer, anti-healing agent, anti-clotting agent, anti-inflammatory, anti-microbial agents, reepitheliating inhibitory agent, nitrous oxide production inhibitory agent, melanogenesis inhibitory agents, dosing agent, emollient, humectant, and the like. To this end, the permeant or bio-compatible filler can also exhibit the functionality of two or more bio-compatible fillers described above. For example, and without limitation, an excipient can also function as an anti-inflammatory agent and/or even a hygroscopic agent. In another aspect, the bio-compatible filler can account for approximately 20 weight % to approximately 80 weight % of the permeant delivery reservoir, including additional amounts as 25 weight %, 30 weight %, 35 weight %, 40 weight %, 45 weight %, 50 weight %, 55 weight %, 60 weight %, 65 weight %, 70 weight %, and 75 weight % of the permeant delivery reservoir, and including any range of weight percentages derived from these values.

As used herein, an anti-healing agent can include, for example, anticoagulants, anti-inflammatory agents, agents that inhibit cellular migration, re-epithelization inhibiting agents, and osmotic agents. Suitable anticoagulants can comprise, for example, heparin having a molecular weight from 3000 to 12,000 daltons, pentosan polysulfate, citric acid, citrate salts, EDTA, and dextrans having a molecular weight from 2000 to 10,000 daltons. Suitable anti-inflammatory agents can comprise, for example, hydrocortisone sodium phosphate, betamethasone sodium phosphate, and triamcinolone sodium phosphate. Suitable agents that inhibit cellular migration can comprise, for example, laminin and/or its related peptides.

As used herein, an osmotic agent can include any biocompatible material, compound, or composition that can generate, in solution, an osmotic pressure greater than about 2000 kilopascals, or mixtures thereof. For example and without limitation, in one aspect the osmotic agent can comprise a biologically compatible salt such as sodium chloride or a neutral compound such as glucose, or a zwitterionic compound such as glycine having a sufficiently high concentration to generate, in solution, a desired osmotic pressure. For example, in one aspect, an osmotic agent, in solution, can generate an osmotic pressure greater than about 2000 kilopascals. In another aspect, an osmotic agent can generate an osmotic pressure greater than about 3000 kilopascals.

To this end, it should be understood that in an alternative aspect, the bio-active agent can also provide the functionality of any one or more bio-compatible fillers described above. For example, and without limitation, a bio-active agent can also exhibit an antihealing effects as set forth above. In particular, in one aspect, the bio-active agent can generate, in solution or suspension, an osmotic pressure greater than approximately 2000 kilopascals such that it is capable of inhibiting the healing process of the at least one formed pathway through the skin of a subject.

As used herein, a hygroscopic agent is intended to include a bio-compatible material, compound or composition having an affinity for subcutaneous fluid such that when present in the permeant, it can enhance the drawing of subcutaneous fluid from the subject into the delivery reservoir. For example, and without limitation, in one aspect a suitable hygroscopic agent that can be used according to the present invention is mannitol. The addition of a hygroscopic filler material may also serves as an attractant to fluid exuding from the treated skin, helping to bring the fluid into the reservoir and in contact with the bioactive agent, while also working to create more diffusion channels from the skin surface side of the reservoir into the body of the reservoir where more bioactive agent can be accessed. Such filler materials should be selected so as to minimize any inhibition of the bioactive agent being delivered into the subject once solubilized and/or suspended.

In one aspect, the biocompatible filler can comprise glycerin, propylene glycol (PG), or a combination thereof. When incorporated as at least a portion of the bio-compatible filler, glycerin and/or propylene glycol can function as one or more of a humectant, hygroscopic agent, emollient, plasticizer, antimicrobial, skin permeation enhancer, and/or anti-irritant. Still further, it should be understood that glycerin and propylene glycol can also effective for use in increasing the release rate of a bioactive agent from a reservoir matrix as described herein and increasing bioactive agent utilization. When used, glycerin and/or propylene glycol are typically present in an amount in the range of from greater than 0.0 % by weight to approximately 5.0 weight % of the permeant delivery reservoir, including amounts of 0.5 weight %, 1.0 weight %, 1.5 weight %, 2.0 weight %, 2.5 weight %, 3.0 weight %, 3.5 weight %, 4.0 weight %, 4.5 weight %, and any range derived from the aforementioned weight percentages.

In another aspect, the biocompatible filler can be selected such that the pH of the fluid it contacts is kept acidic. This can impart an inherent antimicrobial activity against a variety of microorganisms including, without limitation, bacteria, yeast, and mold. In addition, one or more antimicrobial agents can also be added to the polymer film formulation to further enhance the antimicrobial activity of the film.

It will be appreciated upon practicing the present invention that utilizing an anhydrous reservoir design comprising undissolved permeant can improve the shelf stability of the product, reducing the need for refrigeration in many cases. For example, in the case of a protein, peptide, or vaccine antigen, the ability to store the product without refrigeration is an advantage, eliminating the need for refrigeration throughout the distribution network. In the case of vaccine patches, this is an attribute which would allow distribution of vaccines throughout the world without the requirement of a reliable cold chain. The use of an anhydrous formulation can provide still other benefits, including the inherent antimicrobial activity presented by a formulation that does not contain water, and the ability to provide physically smaller reservoirs, as there is no required concentration needed to maintain a stable permeant solution.

As stated above, the at least one hydrophilic permeant is typically disposed or otherwise loaded within the non-biodegradable matrix. To this end, in an exemplary aspect, the delivery reservoir is constructed and arranged such that it has a bottom surface and defines a plurality of conduits therein, wherein at least a portion of the plurality of conduits are in communication with the matrix bottom surface. According to this aspect, the undissolved hydrophilic permeant can be disposed therein at least a portion of the plurality of conduits of the matrix. As such, the exemplified delivery reservoir is thereby adapted to use drawn subcutaneous fluid provided by the fluid loss of the skin to dissolve or suspend at least a portion of the permeant disposed within the matrix thereby enabling diffusion or transport of the permeant into the deeper layers of the skin.

Various mechanisms of transport can effect the dispersion and movement of the undissolved permeant from the reservoir into the skin tissues. In general, but not exclusively, a permeant disposed within the matrix becomes available to the organism upon release by leaving the micro-particulate form and typically going into solution or suspension in the surrounding tissue. Once in solution or suspension, diffusion can provide the transport mechanism for the micro-particulate permeant via the treated outer layers and into or through the viable layers of the skin and into the subject. As the process continues over time, the voids formed by the permeant that leaves the reservoir and moves into the skin form channels penetrating into the body of the reservoir thereby providing additional access to more permeant than was initially present at the surface of the reservoir. Accordingly, by placing the reservoir in communication with at least one formed pathway through a skin layer of a subject, subcutaneous fluid can provide an effective amount or level of hydration to the reservoir to dissolve or suspend the permeant. As such, a relatively high concentration of permeant in solution or suspension can be provided that is also in communication to the viable tissue layers of the skin.

By forming a delivery reservoir according to the present invention, it will be appreciated that it is possible to achieve a relatively high level of permeant utilization not heretofore realized by conventional transdermal delivery devices, systems and methods know for transdermal permeant delivery. Conventional transdermal products rarely utilize more than approximately 30-40% of the bio-active agent present within the reservoir. However, using a conventional residual analysis, the delivery reservoirs of the present invention can, in one aspect, provide a permeant utilization in the range of from 10% to approximately 100%, including such permeant utilizations of at least 15%, 20%, 25%, 30%, 35%, 40%, 45%, 50%, 55%, 60%, 65%, 70%, 75%, 80%, 85%, 90% and 95% and including any range of permeant utilizations derived from these values.

Additionally, it will also be appreciated upon practicing the present invention that a delivery reservoir according to the present invention is capable of maintaining a relatively constant, relatively high chemical potential driving force by continually dissolving or suspending undissolved permeant disposed within the reservoir matrix, thus enabling suspended or dissolved permeant in communication with the at least one formed pathway to remain at or near saturation levels for extended administration periods. By using a non-biodegradable matrix as the permeant carrier, one can effectively 'fill' the space between a plurality of formed pathways over the area of the treated skin site, with an inert, but effectively porous matrix, keeping the required volume of fluid to a minimum. In contrast, conventional methods and devices require a relatively larger quantity of permeant to create the saturation point condition in order to yield the same driving force for the permeant to enter the skin than it does when only the permeant is present in the undissolved solid form reservoir, without any initial fluid. With a traditional pure liquid or gelled aqueous formulation, it takes a much larger quantity of bioactive agent to cover the treated skin site and yield the same saturation level driving force for the bioactive agent to enter the skin than it does when only the bioactive agent is present in the solid form reservoir, without any water other than that presented by the body via the micropores. The functionality of the entire system is in one aspect enabled by the aqueous channels in the skin provided by altering the outermost layers of the skin such that they become permeable during the wear period to a degree sufficient to allow subcutaneous fluid to exit the subject, dissolve or suspend the bioactive agent, and then allow the dissolved or suspended bioactive agent to migrate into the body via these same aqueous channels.

The delivery reservoirs of the instant invention can be manufactured by any conventionally known means for providing a composite reservoir comprised of a solid matrix having at least one undissolved hydrophilic permeant disposed therein. For example, in an exemplified aspect wherein the delivery reservoir comprises a polymer matrix, the polymer and permeant, including any bio-active agent and/or optional filler, can be dry-mixed together using a heated kneading mixer. If the permeant comprises a plurality of components, the plurality of permeant components can, if desired, be premixed to ensure a homogenous permeant composition prior to the mixing of the permeant with the polymeric matrix material. Such permeant pre-blending, if desired, can be performed, for example, on a conventional rotisserie mixer.

The temperature setting of the mixing system should be high enough to allow the particular polymeric material to soften such that it can be kneaded, but not so high as to induce melting of the particular permeant components. Such conditions are of course dependent on the properties of the particular polymeric matrix material and the permeant to be disposed therein. Accordingly, one of skill in the art will be able to readily obtain such operating parameters without requiring undue experimentation. The resulting heat-kneaded mixture can then processed into individual dosage forms of the delivery reservoir comprising, for example, film sheets cut or otherwise configured into any desired shape such as a circular, elliptical, rectangular, square, or any other desired shape.

The permeant delivery reservoir can also be manufactured in any desired thickness, including thicknesses in the range of from approximately 0.01 mm to approximately 30 mm, including such thicknesses as 0.05, 0.1, 0.5, 1.0, 5.0, 10.0, 15.0, 20.0, and 25.0 or even any range of thicknesses derived from these values. For example, the reservoir thickness can be in the range of from 0.01mm to 10.0 mm, or even 0.5 mm to 1.0 mm. To this end, it will be appreciated upon practicing the present invention that the desired thickness can, for example, depend on the particular reservoir components and/or the desired delivery parameters for a given reservoir. For example, in one aspect it may be desired to provide a thicker delivery reservoir film in order to provide a longer administration period. Accordingly, such customization and optimization of the particular delivery reservoir dimensions will be readily obtained by one of skill in the art through no more than mere routine experimentation.

This processing may be accomplished by melt-pressing a quantity of the heat-kneaded admix into a substantially uniform thickness and then using a conventional die cutting method to form the final shape of the delivery reservoir. Alternatively, the processing of the admix can be achieved by extrusion of the heated admix through a die which forms a ribbon of substantial uniform width and thickness, from which the delivery reservoir can be cut either by chopping the ribbon into desired lengths forming, for example, rectangular dosage forms, or die cutting the final dosage form out of the ribbon. In the case of using a die cutting method on the extruded ribbon, the processing machinery can further be configured to recycle the excess 'edges' of the ribbon left after the die cutting procedure, back into the input feed of the mixing/extruding machine, thus achieving a near-zero loss process for mixing the raw components and forming the final dosage form of the reservoir.

Alternatively, a cryo-milled polymeric powder could be mixed with the permeant until a substantially uniform and homogenous distribution of the permeant and polymer is achieved. The resulting mixture can then be hot or cold press formed, or melt extruded into the final desired delivery reservoir shape.

In still another aspect, a conventional solvent casting process can be used wherein the matrix material is dissolved into an organic solvent such as, for example, methylene chloride. The undissolved permeant can then be added to the dissolved polymeric matrix material and the resulting suspension can then poured into reservoir forms having the desired size and shape. The solvent, such as the methylene chloride, can then be evaporated or otherwise removed to provide the permeant delivery reservoir.

As one of skill in the art will appreciate, the relative amounts of bio-active agent(s), filler component(s) and non-biodegradable matrix material can all be adjusted to control the desired transdermal flux rate of the permeant into a subject. For example, the permeant can comprise a filler component, such as a dosing agent, in an amount relative to a predetermined amount of bioactive agent, which can provide a predetermined transdermal dosage of bioactive agent. Alternatively, the permeant composition itself can be present in an amount and composition, relative to a predetermined amount of the solid matrix, which can provide a predetermined rate of transdermal permeant diffusion.

In one aspect, the concentration of undissolved permeant disposed within the anhydrous reservoir is designed to provide the desired statistical probability that upon exposure to a water source, such as the subcutaneous fluid obtained from the micropores in the skin, the water will dissolve or suspend the undissolved permeant such that aqueous channels develop into and through the reservoir, progressively forming throughout the reservoir until the required amount of permeant needed to be delivered to the subject through the micropores has been dissolved or suspended and diffused through these channels, through the micropores and into the subject's skin. By choosing the appropriate ratios, a reservoir can be constructed which insures that substantially all of the permeant in the reservoir will be accessible via these aqueous channels formed by the solvent front as it moves progressively further into the reservoir.

Further, optional excipients or fillers can be included in the reservoir to control the release rate of the bioactive agent, modify the solubility of the bioactive agent in the skin tissues, inhibit or enhance selected physiological phenomena within the affected tissue such as, but not limited to, boosting an immune response, inhibiting an inflammatory response, edema or erythema, maintaining a specified pH range in the tissue, and the like. To this end, by constructing the delivery reservoir to provide a release rate which is more limited than the slowest rate that the skin tissues can absorb the bioactive agent, the system can be made to be extremely repeatable regardless of inter or intra subject variability that typically affect the bioactive agent delivery rate.

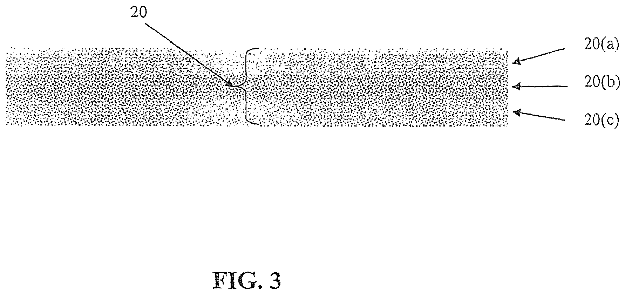

It should also be understood that the device of the present invention is not limited to aspects comprising a single delivery reservoir but further embodies aspects comprising a plurality of delivery reservoirs. For example, as depicted in Figure 3, in one aspect the device of the instant invention can comprise a plurality of delivery reservoirs positioned in a stacked arrangement. As illustrated, a delivery reservoir **20** can comprise, for example and without limitation, three permeant delivery reservoirs, **20(a), 20(b)** and **20(c)** positioned in a stacked arrangement.

Alternatively, a device according to the present invention can comprise plurality of reservoirs positioned in an adjacent or side-by-side relationship. In still another aspect, a device according to the present invention can comprise a combination of a plurality of stacked reservoirs and a plurality of adjacent delivery reservoirs. By providing a multilayered plurality of delivery reservoir, wherein as each layer is sequentially accessed by the solvent front, the predetermined release rate can be varied over a predetermined permeant administration period, thus enabling one of skill in the art to tailor the resulting PK profile of the permeant in the subject. For example, in one aspect, a plurality of delivery reservoirs can be provided wherein at least two reservoirs comprise different dimensional characteristics. In another aspect, at least two reservoirs can be provided, each having a different permeant composition deposited therein. In still another aspect, it is contemplated that a plurality of delivery reservoirs can be provided wherein each of the plurality of reservoirs comprises a different permeant composition.

In still another aspect, a plurality of permeant delivery reservoirs can be arranged to provide a predetermined pattern of pulsatile bioactive agent delivery. This can be done with a completely passive diffusion system wherein the delivery reservoir is constructed with a plurality of reservoir layers, some containing permeant and some not. Thus, as the solvent front moves through the reservoir, bioactive agent will be delivered only during those periods where the layer contains it is at the edge of the solvent front. Similarly, customizing the bioactive agent content in these multiple layers allows the design of a transdermal delivery system which can adjust the influx to be optimal. For example, an insulin delivery system can be constructed to compliment the natural circadian cycles of a subjects glucose metabolism, thus varying the amount of bioactive agent delivered over the dosing period in a programmed fashion to provide better therapy.

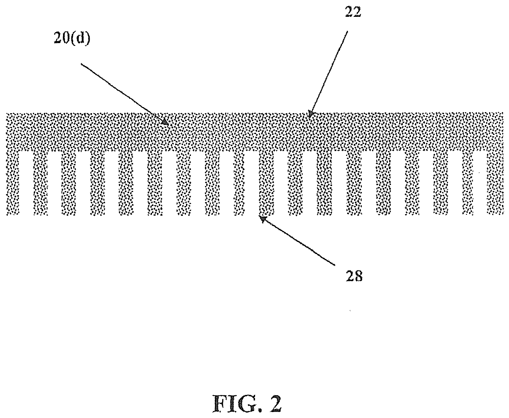

Additional methods for providing permeant release rate control can include altering the physical design of the reservoir, altering the tortuosity of the diffusion paths formed as the solvent front migrates into the reservoir, the choice of anhydrous polymer or other matrix material, or by the addition of specific rate-limiting mechanisms such as a specified membrane or layer within the reservoir. For example, the polymer reservoir can be formed with a specified texture on the skin contact surface said texture designed to increase the surface area of the skin contact surface. By increasing the surface area between the reservoir and the skin, the initial rate of release of bioactive agent into the fluid interface between the patch and the micropores will be greater, resulting in a higher initial flux of the bioactive agent. As the bioactive agent within the reservoir near the textured surface is depleted, and the aqueous porosities penetrating into the polymer reservoir extend further into the reservoir, the flux of the bioactive agent will slow down as the effect of the increased surface area becomes diminished, the further the solvent front moves into the body of the reservoir. Exemplary surface area enhancements can comprise corrugations, perforations, a series of holes extending into the reservoir, either partially through or all the way through or a combination of partial and complete holes, with the partials all at one depth or at an assortment of depths. Essentially, any physical forming of the reservoir that modifies the surface area exposed to the dissolving fluid presented via the micropores, could be used to tailor the flux at various time points during the wear period. Some of the processes useful for forming the reservoir in this manner could be extrusion, stamping, casting, punching, die-cutting, rolling, melting, laser machining, milling, etching or hobbing process, or any combination thereof. These texturing and puncturing of the reservoir in layers can be applied to internal layers that are sandwiched between other layers as well, not just to the layer placed on the surface of the skin. With reference to Figure 2, an exemplary delivery reservoir comprising an enhanced bottom surface area is depicted. As shown, a delivery reservoir **20** can comprise a textured bottom surface **24** wherein the textured surface comprises a series of linear perforations **28.**

It will be appreciated upon practicing the present invention that the reservoir devices described herein can be used to transdermally deliver a permeant for extended administration periods. To that end, a delivery reservoir as described herein can be used to transdermally deliver a permeant to a subject over a predetermined administration period ranging from approximately 1 hour up to approximately 400 hours or more, including administration periods of approximately 5, 10, 50, 100, 150, 200, 250, 300 and 350 hours. Alternatively, the devices of the instant invention can be used to transdermally deliver a predetermined amount of permeant during a predetermined administration period of 6 to 12 hours, 12 to 30 hours, 30 to 50 hours, and even 50 to 80 hours.

To this end, while not intending to be limited by theory, the relatively long administration periods achieved by the devices of the present invention can be a result of the high diffusion gradient resulting from maintaining the dissolved or suspended permeant near the saturation point for extended periods of time. It is further believed that these relatively high osmotic pressure gradients can themselves provide an anti-healing influence on the formed pathway through the opening in the skin layer of a subject further enhancing the ability to achieve extended administration periods. Thus, it should be appreciated that the delivery reservoirs of the present invention can be constructed and arranged to deliver a predetermined level of permeant over virtually any desired administration period.

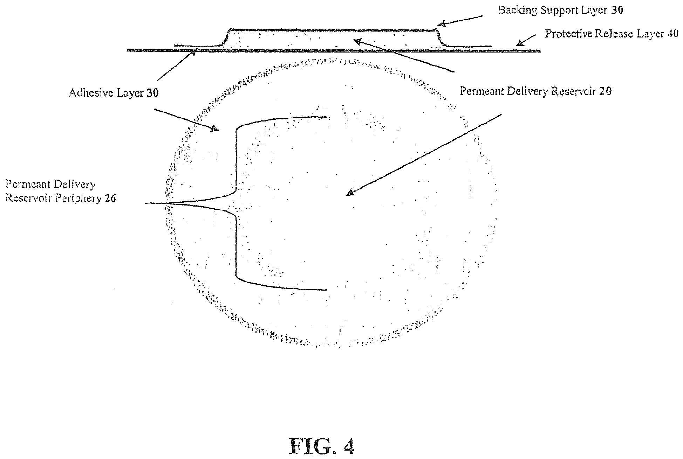

An exemplary device according to one aspect of the present invention is depicted in Figure 4. As illustrated, the exemplary device provides a transdermal patch assembly **10,** comprising a delivery reservoir **20** as previously described herein. The delivery reservoir is constructed and arranged such that it has a top surface **22** and an opposed bottom surface **24.** A backing support layer **30,** having an inwardly facing surface **32** is at least partially connected to the top surface of the delivery reservoir. In one aspect, in order to releasably affix the delivery reservoir to the skin of a subject, the backing support layer can be sized and shaped such that it peripherally extends beyond the delivery reservoir. Further, at least a portion of the inwardly facing surface of the peripherally extending backing support layer can further comprise an adhesive layer **40** deposited thereon. As one of skill in the art will appreciate, the adhesive layer deposited on at least a portion of the backing layer which extends beyond the periphery of the reservoir can provide a peripheral adhesive attachment system.

Alternatively, it is also contemplated that the delivery reservoir can be designed so as to have a skin contact surface tacky enough to releasably adhere directly to the skin of a subject. This can minimize the total size of the patch and reduce the reliance on the peripheral adhesive to maintain sufficient adhesion to adhere the patch to the skin for the duration of the patch wear period (e.g. 1, 2, 3, or 7 days). It will be appreciated upon practice of the invention disclosed herein that such a reservoir can be obtained by, for example, optimizing the percentage of polymer, drug, and/or bio-compatible filler, *i.e.,* excipient, as well as the manufacturing process parameters. Such optimization can be determined by one of skill in the art without the need for undue experimentation.

The backing support layer **30** can in one aspect be at least substantially occlusive. Alternatively, the backing support layer can be at least partially semi-permeable. To this end, in some cases, a semi-permeable backing, such as for example the 3M Tegaderm® product, can provide added user comfort as a vapor permeable backing typically having higher user tolerance for longer wear periods. In addition, the release rate of the drug into the skin can be controlled by controlling the rate of water transport through the film by designing the semi-permeable backing support layer with a specific mean vapor transmission rate (MVTR). In other cases, a more completely occlusive backing may be preferred in order to ensure the maximal hydration of the reservoir from subcutaneous fluid that is accessed from at least one formed pathway beneath the patch assembly as well as from transepidermal water loss through the intact skin surrounding and between the formed pathway(s). Alternatively, the backing can be made totally occlusive to promote hydration of the film and thus contact with the subcutaneous fluid, while the peripheral adhesive can be made semi-permeable to allow better wear characteristics such as better adhesion, and/or lower irritation.

The patch assembly **10** can further comprise a peelable protective release layer **50** sized and shaped to protect at least a portion of the bottom surface of the delivery reservoir from environmental elements until the device is to be used. In one aspect, the protective release layer can be removably secured to at least a portion of peripherally extending backing support layer having the adhesive layer deposited thereon. As will be appreciated, the positioning of the release layer according to this aspect not only provides protection to the bottom surface of the delivery reservoir but can further add a protective layer to the adhesive layer deposited on peripherally extending portion of the backing support layer. The patch assembly comprising the delivery reservoir, backing support layer and, adhesive layer and protective release layer can then placed in an individual pouch and sealed shut.

In use, an exemplary delivery reservoir according to the present invention provides a method for causing the transdermal flux of a permeant into a subject via at least one formed pathway through a skin layer of the subject. In one aspect, the method comprises providing a subject having a transdermal permeant administration site comprising the at least one formed pathway through the skin layer. As used herein, the subject can be any living organism having at least one skin layer capable of transdermal permeant administration. To this end, the subject can be a mammal, such as, for example, a human subject. In an alternative aspect, the subject can be non-mammalian. In still another aspect, the methods and systems of the present invention can be used on a plant.

The transdermal permeant administration site is comprised of at least one formed pathway though a skin layer of the subject. The pathway can be formed by any currently known means for providing a pathway through a skin layer of a subject. To that end, the skin treatment may be some method of forming one or more small, artificial openings, or micropores in the skin within the size range of 1-1000 microns across and 10 to 500 microns deep, which allow fluid communication between the bioactive agent or reservoir and the viable cell layers of the skin beneath the outer most layers of the organisms skin, typically the stratum corneum in a human. These micropores can allow subcutaneous fluid to exude through the micropores to the surface of the skin.

In exemplary aspects, and not meant to be limiting, micropores or pathways in the skin of the subject can be formed by applying thermal poration devices, mechanically puncturing of the skin with micro-needles, lancets or blades, laser ablation, electrical puncturing or ablation, and/or hydraulic jets. Creating pathways by mechanical methods includes use of projections such as solid microneedles or "pyramids" to puncture the skin or scrape tracks or paths through the stratum corneum. The skin treatment may also include, but is not limited to, methods such as the application of acoustic energy or sonication of the skin to increase its permeability, electroporation, tape stripping, abrasive stripping or abrasive treatments, gas jet abrasive treatments, micro-puncturing by the application of high velocity inert particles to the skin via apparatus such as those described by PowderJect Pharmaceutical PLC, chemical treatments, heat treatments, or mechanical treatments to make the skin suitably permeable. Exemplary systems, devices, and methods for forming the desired micropores are discussed in United States Patent Application Nos. 5,885,211, 6,527,716, 6,597,794, 6,611707, 6,692,456, 6,708,060, and 6,711,435 and United States Patent Application Nos. 2004-0220456,2004-0039342, and 2004-003934-3.

After removal of the protective release layer, the patch assembly can then be positioned on the skin of the subject in a manner which at least substantially co-locates the bottom surface of the delivery reservoir over a permeant administration site having at least one formed pathway through a skin layer of the subject, as described herein such that the permeant delivery reservoir comprising an undissolved hydrophilic permeant is in fluid communication with the at least one formed pathway through the skin layer of the subject. Various methods of simplifying the co-location of the active area of the patch to the microporated skin site can be incorporated into an integrated system design such as, for example, a system of visual marks left after the application of the microporation method to allow the user to place the patch in the correct position when these marks are used as reference points. These marks may be formed with a dye or ink or even simply formed by mechanical texture leaving a temporary pattern on the skin; a fold-over co-location system wherein the patch is temporarily attached to the poration system in a fashion which when the poration is accomplished and the poration system is removed from the skin site, a small 'hinge' component is left behind holding the patch such that when the patch is folded over and the hinge is flexed 180 degrees, the needed co-location is ensured; a locator ring of peripheral indicators are left on the skin after the removal of the porator system which provide the needed guides for proper placement of the patch; a fully automated applicator system is used which sequentially applies the poration system, removes it and then applies the patch in a fashion completely transparent and optionally, even hidden, to the user; a fully integrated system is used wherein the porator component is biocompatible, is directly integrated into the skin side of the patch and is designed to allow it to be left in place against the skin under the reservoir after the poration process has be accomplished. Thus, the porator is porous enough to allow the required flux of fluid from the micropores to enter the reservoir and the dissolved or suspended bioactive agent from the reservoir, back around/across the porator and into the micropores.

The permeant delivery reservoir can then be maintained in fluid communication with the at least one formed pathway to draw an effective amount of subcutaneous fluid from the subject through the at least one formed pathway and subsequently transdermally deliver at least a portion of the permeant through the formed pathway at a desired flux. To this end, the subcutaneous fluid drawn through the at least one formed pathway can initiate the process of dissolving and/or suspending at least a portion of the permeant disposed within the reservoir and subsequently can provide a viable diffusion pathway for the permeant to transdermally diffuse back into the subject through the at least one formed pathway in the skin. Once the permeant has been transdermally delivered to a viable skin layer of the subject, the permeant can be active locally or can be taken up by the circulatory system and distributed systemically. For example, in one aspect, the permeant can be taken up by the lymphatic system.

In addition to the passive chemical diffusion based driving forces described herein, it is contemplated that additional permeation enhancers can also be used in combination with the permeant delivery reservoirs of the present invention. For example, and without limitation, the delivery reservoirs of the instant invention can be used in combination with an active force enhancer technology, such as the application of sonic energy, mechanical suction, pressure, or local deformation of the tissues, of which sonophoresis, iontophoresis or electroporation are included.

Still further, additional electromotive forces can also be applied to the permeant in order to enhance the transdermal permeant flux of the permeant through the at least one formed pathway in the skin of the subject. The use of electromotive forces can be particularly useful for transdermal delivery of larger macromolecular agents such as proteins, peptides, and even genes in therapeutical amounts through microporated skin. Moreover, such active delivery modes can in other aspects be used with fewer and/or smaller pathways than are often needed for an equivalent flux via a passive diffusion only system. Thus, in one aspect, the use of active electromotive forces can thereby reduce the volume of skin to be ablated, making the system even less invasive for the user.

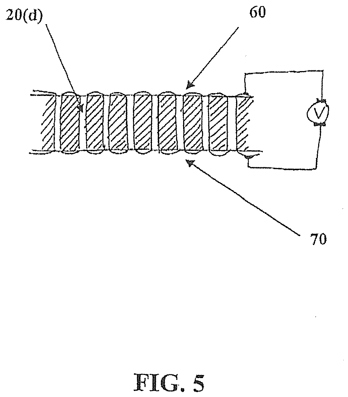

To that end, in one aspect, a permeant delivery reservoir according to the instant invention can be configured to provide an electro-osmotic-pump (EOP) assembly. According to this aspect, and as depicted in Figure 5, a microporated delivery reservoir **20(d)** having a top surface and an opposed bottom surface, can further comprise an assembly of one or more first electrodes **60** positioned in electrical communication with the top surface and an assembly of one or more second electrodes **70** positioned in electrical communication with the bottom surface. The electrode assemblies can be provided by any conventional electrode deposition techniques know to one of skill in the art, such as, for example, sputtering, electrodeposition, or electro-less deposition. A complete circuit can then be created by placing the first and second electrode assemblies in selective or controllable electrical communication with a voltage or current source (V). A steady application of a properly polarized electrical field to the permeant within the microporated reservoir can induce a build up of permeant in the vicinity of the openings of the microporated reservoir, thus providing a relative boost to the diffusion gradient driven transdermal delivery into a subject.

In still another aspect, an electro-osmotic-pump assembly according to the present invention can further comprise a third or counter electrode remotely positioned from the delivery reservoir and adapted to be positioned in electrical communication with the skin of a subject. The incorporation of a third, or counter electrode, can enable the application of an electromotive force capable of enhancing the movement of the permeant from the bottom surface of the microporated delivery reservoir laterally to foci coincident with the at least one formed pathway in the skin of the subject. As will be appreciated, this aspect of the invention can provide additional transdermal flux efficiency since there will be essentially zero flux through the intact portions of the skin which still have the undisrupted stratum corneum layer and do not have a formed pathway open to the viable layers of the skin.

In use, a three-electrode assembly as described above can be operated according to a selective on-off cycling of the various electrode assemblies within the electro-osmotic pump assembly. For example, in a first electro-osmotic pump cycle, the electro-osmotic pump (EOP) can be activated by completing a circuit between the first and second electrode assemblies in order to create a relatively high concentration of the bio-active agent in the proximity of the microporous openings in the bottom surface of the delivery reservoir. During a second electro-transport cycle, one or both of the first and second EOP electrode assemblies can be charged with the same polarity as the net charge on the particular bio-active agent to be transdermally delivered. The third electrode assembly, which can be positioned remotely from the delivery reservoir and in communication with the surface of the skin, can then be operated as a counter electrode. In this electro-transport mode, the electro-repulsive force exerted on the bioactive agent can actively drive the bioactive agent into the micropores of the subject.

Of course, it should be appreciated that this electro-transport mode (ETM) and the electro-osmotic-pump mode (EOP) can be modulated in an on-off manner, or in any level between off and maximum intensity. By keeping the amount and duration of the ETM within certain exemplary limits, such as, for example, 10 ms on and 50 ms off, the average current which will flow through the skin tissues of a subject during ETM can be kept to a low enough level that any shifts in local pH can be neutralized during the off-time of the ETM by the normal micro-fluidic action within the skin tissues and the natural diffusion of ions when no electric field is present. As will be appreciated by one of skill in the art, this can work to establish uniform concentration of all mobile species, thus bringing the pH back to its normal physiological state. As such, this modulation of on-time to off-time of the ETM can also eliminate irritation due to a disruption of the normal pH of the skin tissues.

It should be understood that the specific duty cycles of the EOP mode or cycle and the ETM mode or cycle can depend on the particular permeant to be transdermally delivered and the current levels applied to both the EOP and ETM. Whereas a rough calculation can be made that will ensure the pH of the viable tissues stays within some predetermined boundary, in practice, these duty cycles can be determined experimentally by simply placing a small pH sensor under the patch to monitor the effects of different duty cycles. A further feature of this invention would be to incorporate a pH sensing element into the patch and use the output generated by it as a feedback signal to the system controller such that a closed-loop control circuit is implemented which ensures that the pH is held within the programmed boundaries, regardless of subject-to-subject variations in local skin physiology, environmental factors, or other forces which may affect the local environment.

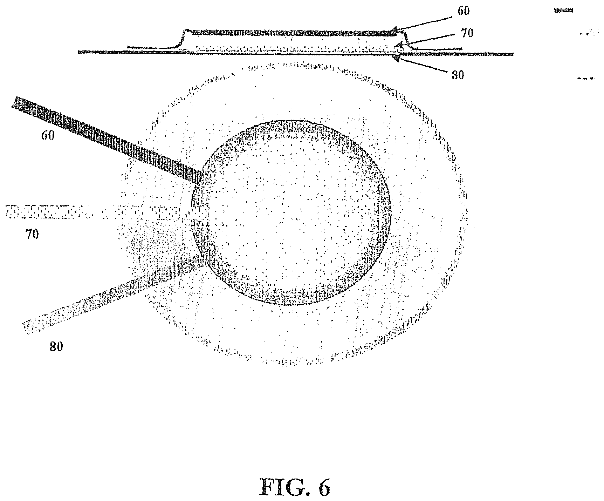

With reference to Figure 6, an exemplary patch assembly further comprising a three-electrode osmotic pump assembly is depicted. As illustrated, the exemplary device comprises a transdermal patch assembly 10, comprising a microporated delivery reservoir **20(d)** as previously described herein. The delivery reservoir is constructed and arranged such that it has a top surface **22** and an opposed bottom surface **24.** A backing support layer **30,** having an inwardly facing surface **32** is at least partially connected to the top surface of the delivery reservoir. The microporated delivery reservoir **20(d)** comprises a top surface **22** and an opposed bottom surface **24.** A first electrode assembly **60** is positioned in electrical communication with the top surface and an second electrode assembly **70** is positioned in electrical communication with the bottom surface. A third or counter electrode **80** is remotely positioned from the delivery reservoir and adapted to be positioned in electrical communication with the skin of a subject. A complete circuit can then be created between at least any two of the first, second and third electrodes by placing at least two of the first, second and third electrode assemblies in selective or controllable electrical communication with a voltage or current source (not illustrated).

### EXAMPLES

The following examples are put forth so as to provide those of ordinary skill in the art with a complete disclosure and description of how the devices, systems and methods claimed herein are made, performed and evaluated. These examples are intended to be purely exemplary of the invention and are not intended to limit the scope of what the inventors regard as their invention. Efforts have been made to ensure accuracy with respect to numbers (e.g., amounts, temperatures, etc.); however, some errors and deviations may have occurred. Unless indicated otherwise, parts are parts by weight, temperature is degrees C or is at ambient temperature, and pressure is at or near atmospheric.

### Example 1: Preparation of Permeant Delivery Reservoir Comprising Hydromorphone HCl as Bioactive agent and Propylene Glycol as a biocompatible filler.

An exemplary permeant delivery reservoir comprising hydromorphone HCl as the bioactive agent could be prepared according to the exemplary procedures set forth below.

The reservoir can be prepared by charging approximately 1140 mg of ethylene vinyl acetate comprised of approximately 40 % vinyl acetate component, approximately 1330 mg of hydromorphone HCl; approximately 1330 mg of Mannitol and approximately 200 mg of propylene glycol can in a vial and allowing the mixture to blend overnight. The vial can then be heated in a silicone oil bath to a temperature in the range of approximately 80°C to 100°C while continuously mixing with a spatula. After the mixture achieves a dough-like consistency the mixture can then be transferred to a backing film such as the Scotchpak backing available from 3M®.

Once deposited on the backing material, the dough-like reservoir material can be compressed between the backing layer and a protective release liner layer (such as the 1521 single-sided polyethylene film, also available from 3M®) to provide a reservoir having a desired thickness. After the reservoir material has cooled, the resulting film can then be cut to provide a patch having a reservoir surface area of, for example, approximately 1 cm². A reservoir prepared according to the foregoing procedure can, for example, comprise a concentration of bioactive agent of approximately 35 mg hydromorphone HCl per patch. Prior to applying the exemplary reservoir onto a test subject, the protection release layer would first be removed to expose the bottom surface of the reservoir.

### Example 2: Preparation of Permeant Delivery Reservoir Comprising Insulin as Bio-active agent

An exemplary permeant delivery reservoir comprising lyophilized insulin as the bioactive agent can be prepared according to the exemplary procedures set forth below.

Lyophilized insulin can first be prepared by dissolving approximately 40 mg of raw insulin material with 40 mg of mannitol, approximately 3.48 mg of arginine and approximately 16 mg of trehalose in approximately 0.9 mL of distilled water. If desired, the pH can then be adjusted with IN sodium hydroxide or with approximately 0.1N hydrochloric acid to achieve a pH in the range of approximately 8.8-9.0. The resulting solution can then be frozen at a temperature of approximately -80 °C and then subsequently dried under vacuum for at least approximately 16 hours to provide the lyophilized insulin.

A permeant reservoir comprising lyophilized insulin can then be prepared by a solvent casting process. To this end, approximately 350 mg of ethylene vinyl acetate co-polymer can be dissolved in approximately 4 mL of methylene chloride under vigorous shaking. Approximately 1225 mg of sieved mannitol and approximately 174 mg of sieved lyophilized insulin can then be added into the EVA and methylene chloride solution. The resulting suspension can then be stirred at 1200 rpm for approximately 10 minutes followed by a tube rolling for approximately 30 additional minutes.

After mixing, the resulting suspension can then be poured onto a Scotchpak® backing film and drawn to a 50 mil thickness using a micro film applicator such as the 50 mil applicator available from Paul N. Gardner Co., Inc. The drawn film can then be dried in a fume hood at ambient temperature and pressure for a period of time in the range of 3 to 16 hours. The dried film can then be stored in a refrigerated dessicator until it is to be used.

### Example 3: Preparation of Permeant Delivery Reservoir Comprising Hydromorphone HCl as Bioactive agent and Propylene Glycol as a biocompatible filler.

An exemplary permeant delivery reservoir comprising hydromorphone HCl as the bioactive agent and propylene glycol as a bio-active filler can be prepared according to the exemplary procedures set forth below.

Initially, bulk Hydromorphone HCl and mannitol can be sieved using a 200 mesh sieve before use. The reservoir can then be prepared by charging approximately 9975 mg of hydromorphone HCl and approximately 9975 mg of Mannitol into a vial blending the mixture for at least 4 hours. Approximately 8550 mg of ethylene vinyl acetate comprised of approximately 40 % vinyl acetate component and 1500 mg of propylene glycol can be added to the blended mix of hydromorphone HCl and Mannitol. The charged materials can be continuously stirred and heated in temperature controlled container to a temperature in the range of approximately 80 °C to 120 °C. After the mixture achieves a dough-like consistency, the mixture can then be transferred to a backing film such as the Scotchpak backing available from 3M®.

Once deposited on the backing material, the dough-like reservoir material can be compressed between the backing layer and a protective release liner layer (such as the 1521 single-sided polyethylene film, also available from 3M®) to provide a reservoir having a desired thickness. After the reservoir material has cooled, the resulting film can then be cut to provide a patch having a reservoir surface area of, for example, approximately 1 cm². A reservoir prepared according to the foregoing procedure can, for example, comprise a concentration of bioactive agent of approximately 21 mg hydromorphone HCl per patch. Prior to applying the exemplary reservoir onto a test subject, the protection release layer would first be removed to expose the bottom surface of the reservoir.

### Example 4: Preparation of Permeant Delivery Reservoir Comprising Hydromorphone HCl as Bioactive agent and 1% Glycerin as a biocompatible filler.

An exemplary permeant delivery reservoir comprising hydromorphone HCl as the bioactive agent and 1.0 weight % glycerin as a bio-active filler can be prepared according to the exemplary procedures set forth below.

Again, bulk Hydromorphone HCl and mannitol can be sieved using a 200 mesh sieve before use. The reservoir can be prepared by charging approximately 10575 mg of hydromorphone HCl and approximately 10575 mg of Mannitol into a vial and blending the mixture for at least 4 hours. Approximately 8550 mg of ethylene vinyl acetate comprised of approximately 40 % vinyl acetate component and 300 mg of glycerin are added to the blended mix of hydromorphone HCl and Mannitol. The charged materials can be continuously stirred and heated in a temperature controlled container to a temperature in the range of approximately 80 °C to 120 °C. After the mixture achieves a dough-like consistency, the mixture can then be transferred to a backing film such as the Scotchpak backing available from 3M®.

Once deposited on the backing material, the dough-like reservoir material can be compressed between the backing layer and a protective release liner layer (such as the 1521 single sided polyethylene film, also available from 3M®) to provide a reservoir having a desired thickness. After the reservoir material has cooled, the resulting film can then be cut to provide a patch having a reservoir surface area of, for example, approximately 1 cm². A reservoir prepared according to the foregoing procedure can, for example, comprise a concentration of bioactive agent of approximately 23 mg hydromorphone HCl per patch. Prior to applying the exemplary reservoir onto a test subject, the protection release layer would first be removed to expose the bottom surface of the reservoir.

### Example 5: Preparation of Permeant Delivery Reservoir Comprising Hydromorphone HCl as Bioactive agent and 0.5% Glycerin as a biocompatible filler.

An exemplary permeant delivery reservoir comprising hydromorphone HCl as the bioactive agent and 0.5 weight % glycerin as a bio-active filler can be prepared according to the exemplary procedures set forth below.

To prepare the reservoir, bulk Hydromorphone HCl and mannitol can first be sieved using a 200 mesh sieve before use. The reservoir can then be prepared by charging approximately 10650 mg of hydromorphone HCl and approximately 10650 mg of Mannitol into a vial and allowing the mixture to blend for at least 4 hours. Approximately 8550 mg of ethylene vinyl acetate comprised of approximately 40 % vinyl acetate component and 150 mg of glycerin can be added to the blended mix of hydromorphone HCl and Mannitol. The charged materials can be continuously stirred and heated in temperature controlled container to a temperature in the range of approximately 80 °C to 120 °C. After the mixture achieves a dough-like consistency the mixture can then be transferred to a backing film such as the Scotchpak backing available from 3M®.

Once deposited on the backing material, the dough-like reservoir material can be compressed between the backing layer and a protective release liner layer (such as the 1521 single-sided polyethylene film, also available from 3M®) to provide a reservoir having a desired thickness. After the reservoir material has cooled, the resulting film can then be cut to provide a patch having a reservoir surface area of, for example, approximately 1 cm². A reservoir prepared according to the foregoing procedure can, for example, comprise a concentration of bioactive agent of approximately 23.5 mg hydromorphone HCl per patch. Prior to applying the exemplary reservoir onto a test subject, the protection release layer would first be removed to expose the bottom surface of the reservoir.

### Example 6: Preparation of Permeant Delivery Reservoir Comprising Hydromorphone HCl as Bioactive agent without Glycerin or Propylene Glycol as a bio-compatible filler

An exemplary permeant delivery reservoir comprising hydromorphone HCl as the bioactive agent and without glycerin or propylene glycol as a bio-active filler can be prepared according to the exemplary procedures set forth below.

To prepare the reservoir, bulk Hydromorphone HCl and mannitol can be sieved using a 200 mesh sieve before use. The reservoir can then be prepare by charging approximately 10725 mg of hydromorphone HCl and approximately 10725 mg of Mannitol into a vial and allowing the mixture to blend for at least 4 hours. Approximately 8550 mg of ethylene vinyl acetate comprised of approximately 40 % vinyl acetate component can be added to the blended mix of hydromorphone HCl and Mannitol. The charged materials can be continuously stirred and heated in temperature controlled container to a temperature in the range of approximately 80 °C to 120 °C. After the mixture achieves a dough-like consistency the mixture can then be transferred to a backing film such as the Scotchpak backing available from 3M®.

Once deposited on the backing material, the dough-like reservoir material can be compressed between the backing layer and a protective release liner layer (such as the 1521 single-sided polyethylene film, also available from 3M®) to provide a reservoir having a desired thickness. After the reservoir material has cooled, the resulting film can then be cut to provide a patch having a reservoir surface area of, for example, approximately 1 cm². A reservoir prepared according to the foregoing procedure can, for example, comprise a concentration of bioactive agent of approximately 21 mg hydromorphone HCl per patch. Prior to applying the exemplary reservoir onto a test subject, the protection release layer would first be removed to expose the bottom surface of the reservoir.

### Example 7: Preparation of Permeant Delivery Reservoir Comprising 10 % Fentanyl Citrate as Bioactive agent.

An exemplary permeant delivery *reservoir* comprising 10% fentanyl citrate *as* the bioactive agent can be prepared according to the exemplary procedures set forth below.

To prepare the reservoir, mannitol is sieved using a 200 mesh sieve before use. The reservoir can then be prepare by charging approximately 3000 mg of Fentanyl citrate and approximately 18450 mg of Mannitol into a vial and allowing the mixture to blend for at least 6 hours. Approximately 8550 mg of ethylene vinyl acetate comprised of approximately 40 % vinyl acetate component can be added to the blended mix of Fentanyl citrate and Mannitol. The charged materials can be continuously stirred and heated in a temperature controlled container to a temperature in the range of approximately 80 °C to 120 °C. After the mixture achieves a dough-like consistency, the mixture can then be transferred to a backing film such as the Scotchpak backing available from 3M®.

Once deposited on the backing material, the dough-like reservoir material can be compressed between the backing layer and a protective release liner layer (such as the 1521 single-sided polyethylene film, also available from 3M®) to provide a reservoir having a desired thickness. After the reservoir material has cooled, the resulting film can then be cut to provide a patch having a reservoir surface area of, for example, approximately 1 cm². A reservoir prepared according to the foregoing procedure can, for example, comprise a concentration of bioactive agent of approximately 3.8 mg Fentanyl citrate per patch. Prior to applying the exemplary reservoir onto a test subject, the protection release layer would first be removed to expose the bottom surface of the reservoir.

### Example 8: Preparation of Permeant Delivery Reservoir Comprising 5 % Fentanyl Citrate as Bioactive agent.

An exemplary permeant delivery reservoir comprising 5 % fentanyl citrate as the bioactive agent can be prepared according to the exemplary procedures set forth below.

To prepare the reservoir, mannitol can first be sieved using a 200 mesh sieve before use. The reservoir can then be prepare by charging approximately 1500 mg of Fentanyl citrate and approximately 19950 mg of Mannitol into a vial and allowing the mixture to blend for at least 6 hours. Approximately 8550 mg of ethylene vinyl acetate comprised of approximately 40 % vinyl acetate component can be added to the blended mix of Fentanyl citrate and Mannitol. The charged materials can be continuously stirred and heated in a temperature controlled container to a temperature in the range of approximately 80 °C to 120 °C. After the mixture achieves a dough-like consistency, the mixture can then be transferred to a backing film such as the Scotchpak backing available from 3M®.

Once deposited on the backing material, the dough-like reservoir material can be compressed between the backing layer and a protective release liner layer (such as the 1521 single-sided polyethylene film, also available from 3M®) to provide a reservoir having a desired thickness. After the reservoir material has cooled, the resulting film can then be cut to provide a patch having a reservoir surface area of, for example, approximately 1 cm². A reservoir prepared according to the foregoing procedure can, for example, comprise a concentration of bioactive agent of approximately 1.8 mg Fentanyl citrate per patch. Prior to applying the exemplary reservoir onto a test subject, the protection release layer would first be removed to expose the bottom surface of the reservoir.

### Permeant Reservoir Performance Studies

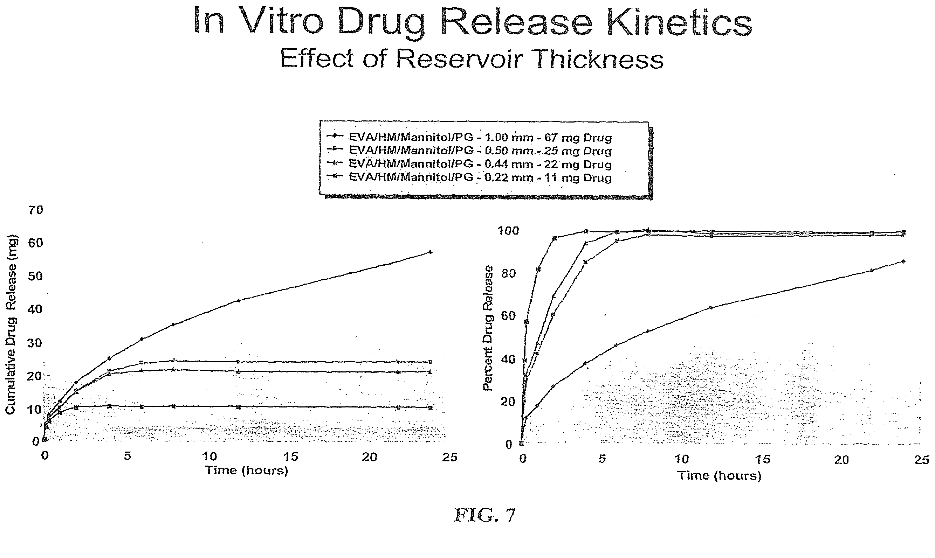

In order to evaluate the efficacy of the delivery reservoirs of the instant invention, several tests were performed using permeant delivery reservoirs similar to those that could be made according to procedures set forth in Examples 1 thru 8. The results of the various tests evaluating the permeant delivery reservoirs of the instant invention are reported in Figures 7 through 28 and are briefly discussed below.

Figure 7 reports by comparison, the effect of permeant delivery reservoir thickness on the in vitro drug release kinetics for various permeant delivery reservoirs of the present invention. Four permeant delivery reservoirs were prepared according to the present invention. The four reservoir matrices each comprised ethylene vinyl acetate copolymer (EVA). The permeant formulations disposed within the EVA reservoirs comprised hydromorphone HCl (HM) as the bioactive agent and mannitol and propylene glycol (PG) as a filler component and were approximately 1.44 cm² in area. The first reservoir had a thickness of approximately 1.00 mm and comprised approximately 67 mg of hydromorphone. The second reservoir had a thickness of approximately 0.50 mm and comprised approximately 25 mg of hydromorphone HCl. The third reservoir had a thickness of approximately 0.44 mm and comprised approximately 22 mg of hydromorphone. The fourth reservoir had a thickness of approximately 0.22 mm and comprised approximately 11 mg of hydromorphone HCl.

In vitro tests using each of the four reservoirs were conducted for an administration period of approximately 24 hours. Using conventional means for analysis, the cumulative hydromorphone HCl release and relative percentage of hydromorphone HCl release for each of the four permeant delivery reservoirs over the 24 hour administration period are reported by the plots depicted in Figure 7.

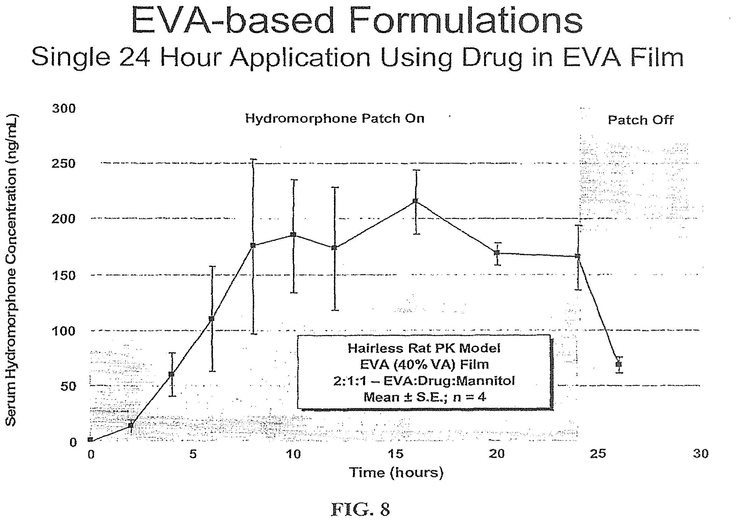

Figure 8 reports the mean pharmacokinetic profile (PK profile) for an exemplary permeant delivery reservoir device according to the present invention that was tested on the abdomen region of four different hairless rat subjects. The permeant reservoir was a film having a thickness of approximately 1.4 millimeters and comprised 50 weight percent of an ethylene vinyl acetate copolymer having approximately 40% vinyl acetate component as the matrix material. The permeant composition comprised 25 weight percent hydromorphone HCl (relative to the total weight percent of the permeant reservoir) as the bio-active agent and 25 weight percent mannitol (relative to the total weight of the permeant reservoir) as additional filler component. The mean serum hydromorphone concentration in the hairless rats as a function of a 24-hour administration period is reported in Figure 8.

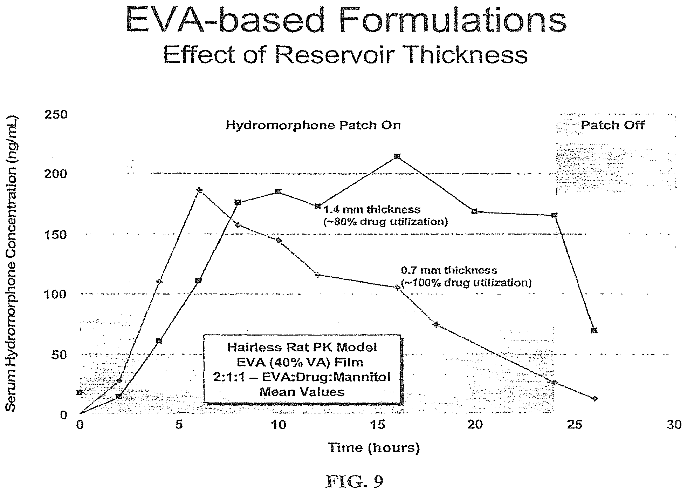

Figure 9 illustrates a comparison of the pharmacokinetic profile data reported in Figure 8 against the pharmacokinetic profile of a similar permeant delivery reservoir having a thickness if approximately 0.7 mm. As illustrated, the permeant reservoir having a thickness of approximately 1.4 mm exhibited a mean hydromorphone HCl utilization of approximately 80% whereas the reservoir having a thickness of approximately 0.7 mm exhibited a mean hydromorphone utilization of approximately 100%.

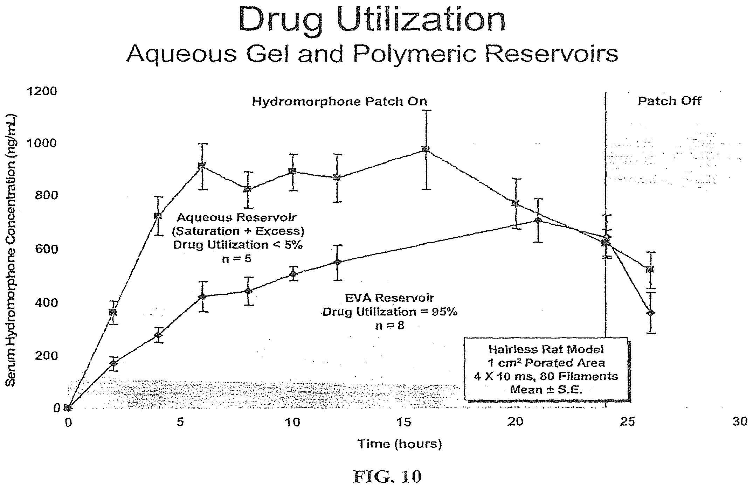

Figure 10 illustrates a comparison of mean pharmacokinetic profiles for a hydromorphone containing aqueous reservoir and for a hydromorphone containing permeant reservoir according to the present invention. The aqueous reservoir comprised hydromorphone in a 4% HPMC (hydroxypropylmethyl cellulose) gel. The permeant reservoir exemplary of the instant invention comprised approximately 40 wt. % EVA (having 40% vinyl acetate component), approximately 30 wt. % mannitol, and approximately 30 wt. % hydromorphone. The respective permeant reservoirs were each tested on 8 hairless rat test subjects by applying the reservoir to a 1 cm² microporated administration site. The administration site was provided on the skin of the hairless rat subjects by thermal poration using an apparatus having an array of 80 thermal poration filaments, such as the PassPort™ thermal poration system from Altea Therapeutics. The thermal poration apparatus was operated 4 times in 10 millisecond pulses. As reported, the aqueous reservoir provided a mean hydromorphone utilization of approximately less than 5% whereas the mean hydromorphone utilization of the permeant reservoir according to the present invention was approximately 95%.

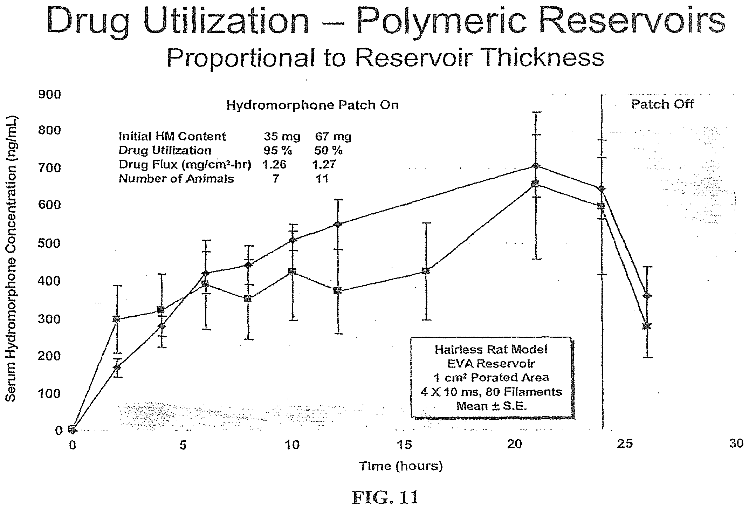

Figure 11 reports a comparison of mean pharmacokinetic profiles for two different permeant delivery devices according to the instant invention as a function of the permeant reservoir thickness. The top curve represents data resulting from a reservoir having a composition of approximately 35 mg of hydromorphone and comprising approximately 28.5 wt.% EVA, 33.25 wt.% mannitol, 5 wt.% propylene glycol and 33.25 wt.% hydromorphone. The bottom curve represents data resulting from a similar reservoir having approximately 67 mg of hydromorphone and having a thickness approximately twice that of the reservoir comprising 35 mg of hydromorphone. As reported, the thicker reservoir comprising approximately 67 mg of hydromorphone HCl provided a mean utilization of approximately 50% when tested upon 11 hairless rat subjects. In contrast, the reservoir having a smaller thickness and comprising approximately 35 mg of hydromorphone HCl provided a mean utilization of approximately 95% when tested on 7 hairless rat subjects.

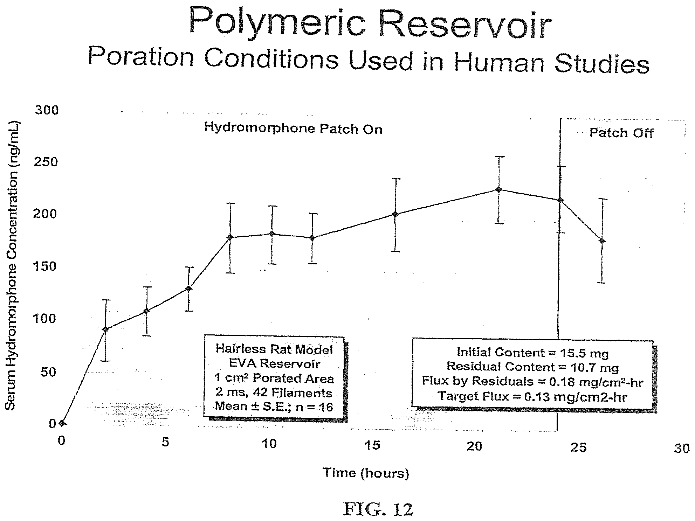

Figure 12 reports the mean pharmacokinetic profile (PK profile) for an exemplary permeant delivery reservoir device according to the present invention that was tested on the abdomen region of sixteen hairless rat subjects. The permeant reservoir was a film reservoir that was produced according to a method similar to that of Example 1, having a thickness of approximately .22 millimeters and comprising approximately 15.5 mg of hydromorphone HCl. The permeant reservoirs were tested on the 16 hairless rat test subjects by applying the reservoirs to a 1 cm² microporated administration site. The administration site was provided on the skin of the hairless rat subjects by thermal poration using an apparatus having an array of 42 thermal poration filaments, such as the PassPort™ thermal poration system from Altea Therapeutics. The 42 filament array was operated for a 2 millisecond pulse. As reported in Figure 12, at the conclusion of a 24-hour administration period, the mean residual hydromorphone content of the delivery reservoir was approximately 10.7 mg of hydromorphone. Further, the reservoir provided a mean flux of approximately 0.18 mg/cm²-hour with the targeted flux being approximately 0.13 mg/cm²-hour.

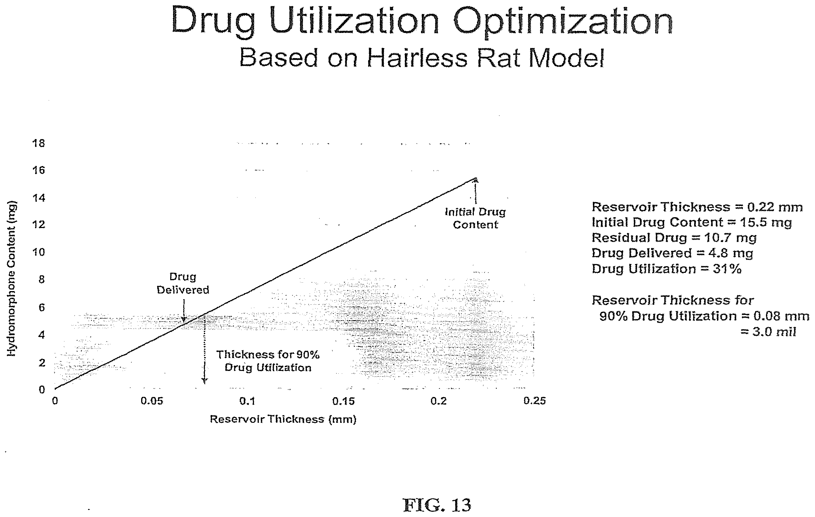

Utilizing the mean data obtained and reported in Figure 12, Figure 13 reports an exemplary ability to optimize the drug utilization of a given permeant delivery reservoir. To this end, the data reported in Figure 12 indicates that the permeant reservoirs tested therein provided a mean hydromorphone utilization of approximately 31%. Using a linear extrapolation of this data, it can be calculated that by providing a reservoir having a thickness of approximately 0.08 mm, a reservoir could be provided that exhibits a mean utilization of approximately 90%.

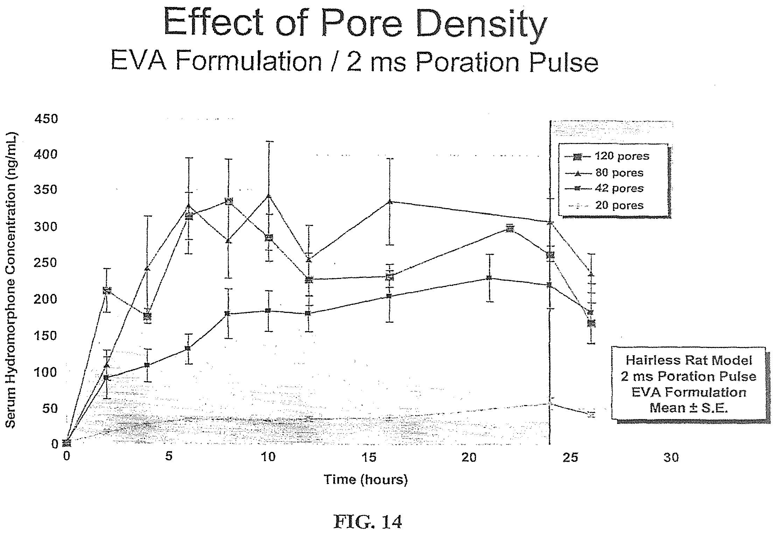

Figure 14 reports the effect of pore density within the administration site on the mean pharmacokinetic profile of a permeant reservoir according to the present invention. As illustrated, altering pore density can, in one aspect, result in differing flux. As also illustrated, in one aspect, an increase in pore density can be used to provide a higher flux.

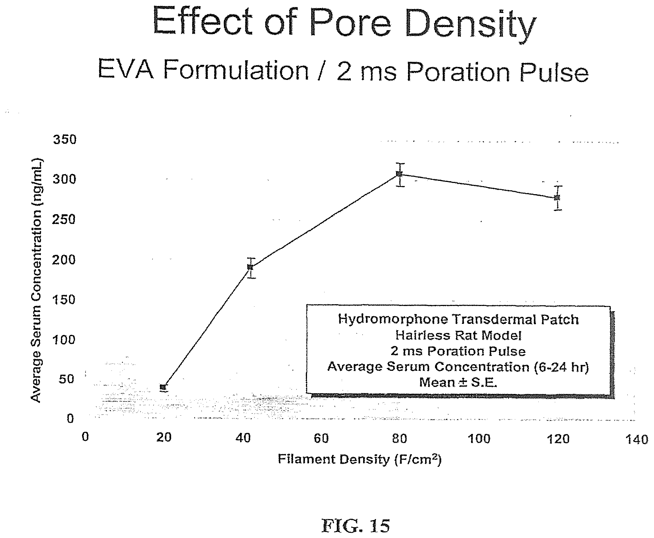

Figure 15 reports the effect of pore density on the average hydromorphone serum concentration during a 6-24 hour administration period. As reported, the mean serum concentration can be expressed as a function of the filament density used to provide the thermally porated administration site. Thus it can be seen that another means for optimizing and/or customizing the desired delivery performance of a given permeant reservoir, in one aspect, comprises selecting a particular density of micropores in a given permeant administration site.

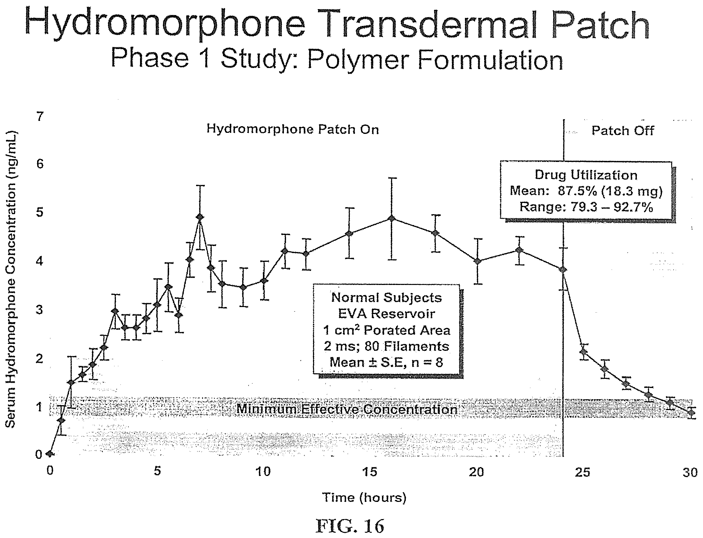

Figure 16 reports the mean serum hydromorphone concentration among 8 normal test subjects as a function of time during a 24-hour administration period that comprised administering hydromorphone to the test subjects using a permeant reservoir provided in accordance to the present invention. As indicated, the permeant reservoirs of the present invention can, in one aspect, provide a mean utilization of approximately 87.5%, which in this example, resulted from a range of utilizations of from approximately 79.3% to approximately 92.7%.

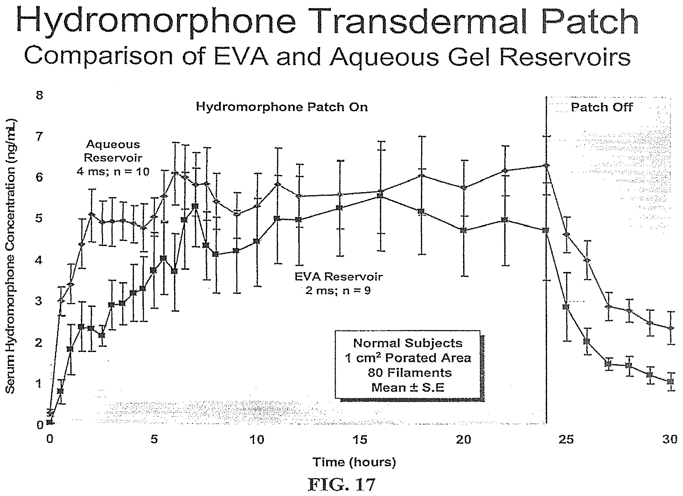

Figure 17 reports, by comparison, the mean pharmacokinetic profile of hydromorphone delivered to nine normal human test subjects using a reservoir described herein against the mean pharmacokinetic profile of hydromorphone delivered to ten normal human test subjects using an aqueous reservoir containing hydromorphone at or near the saturation point.

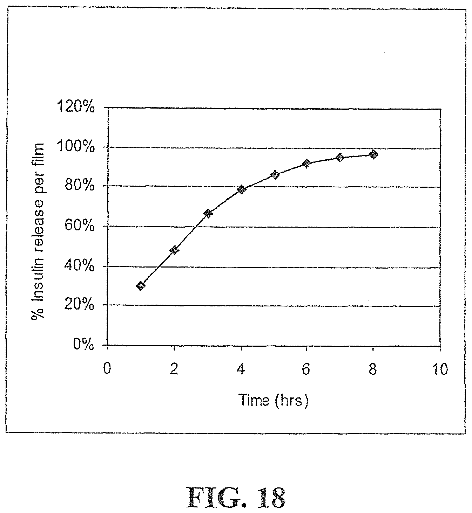

Figure 18 reports the mean cumulative insulin release kinetics for a permeant delivery reservoir that could be provided according to Example 2. The permeant reservoir was tested on four subjects during a 24-hour administration period. The delivery reservoir comprised approximately 20 weight % of an ethylene vinyl acetate co-polymer that was comprised of approximately 40 % vinyl acetate component. Disposed within the EVA matrix was 20 weight % insulin relative to the total weight of the delivery reservoir, 52 weight % mannitol relative to the total weight of the delivery reservoir and 8 weight % trehalose relative to the total weight of the delivery reservoir.

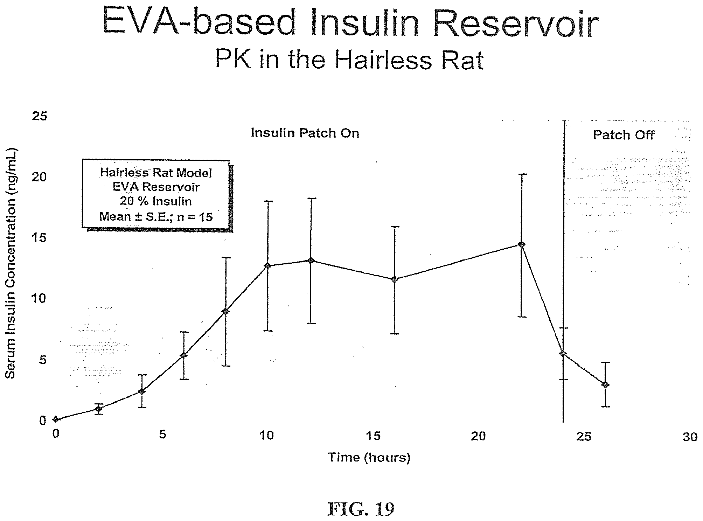

Figure 19 reports the mean serum insulin concentration levels from 15 hairless rat subjects that were administered insulin via a delivery reservoir described herein. To that end, the data reported in Figure 19 illustrates the ability of a delivery reservoir of the present invention, comprising insulin as a bioactive agent, to transdermally deliver an effective amount of the insulin to a subject over a 24-hour administration period.

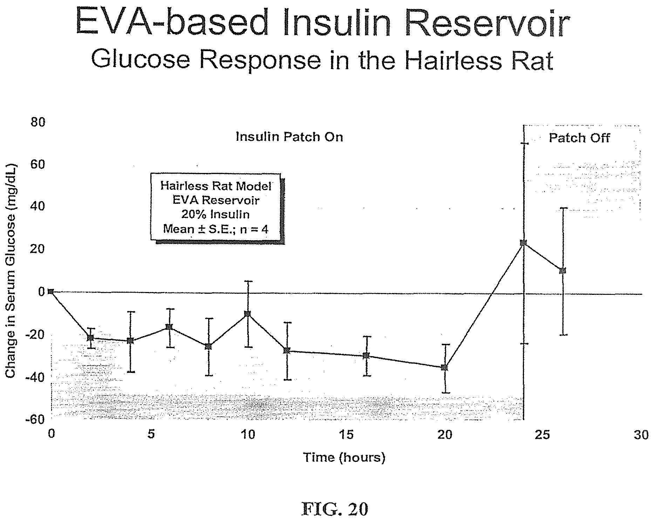

Figure 20 reports the mean changes in serum glucose concentrations among 4 hairless rat subjects that were administered insulin transdermally via a permeant delivery reservoir described herein. To that end, the data reported in Figure 20 once again indicates the successful transdermal delivery of insulin using a reservoir described herein, as characterized by the corresponding changes in serum glucose concentrations.

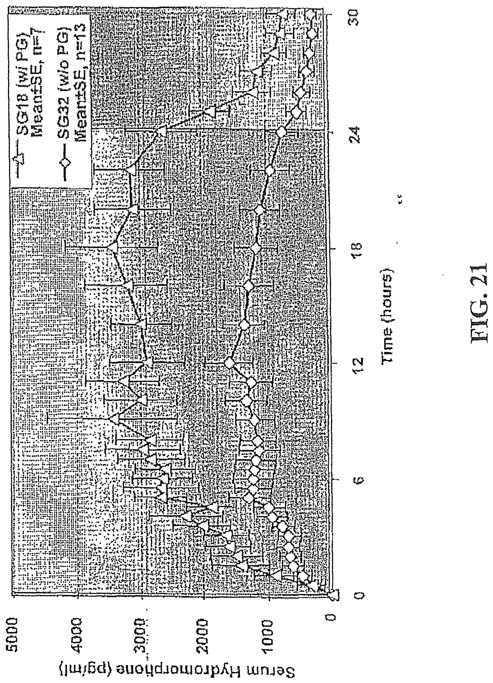

Figure 21 reports, by comparison, the enhancing effect propylene glycol can have on the steady-state serum hydromorphone levels in a clinical pharmacokinetic profile study involving healthy human test subjects. A series of film permeant delivery reservoirs comprising hydromorphone HCl as the bio-active agent and propylene glycol as a bio-compatible filler were prepared, having the formulation 33.25% (w/w) Hydromorphone hydrochloride, 28.5% (w/w) Ethylene vinyl acetate (40% VA) film, 33.25% (w/w) Mannitol, and 5% (w/w)Propylene glycol. Similarly, a series of film permeant delivery reservoirs comprising hydromorphone HCl as the bio-active agent but with out propylene glycol as a biocompatible filler were also prepared, having the formulation 35.75% (w/w) Hydromorphone hydrochloride, 28.5% (w/w) Ethylene vinyl acetate (40% VA) film, and 35.75% (w/w) Mannitol.

Permeant delivery reservoirs without the propylene glycol were tested on a 1 cm² microporated administration site prepared on the upper arm region of thirteen healthy human test subjects for an administration period of 24 hours. Likewise, permeant delivery reservoirs with the propylene glycol were tested on a 1 cm² microporated administration site prepared on the upper arm region of seven healthy human *test* subjects, also for an administration period of 24 hours. The administration sites were provided on the skin of the test subjects by thermal poration using an apparatus having an array of 120 thermal poration filaments, such as the PassPort™ thermal poration system from Altea Therapeutics, Tucker, Georgia, USA. The filament array was operated for a 2 millisecond pulse. As shown in Figure 21, the formulation without propylene glycol resulted in mean steady state serum levels of hydromorphone that were approximately 2.5 times lower than those obtained with the formulation comprising propylene glycol.

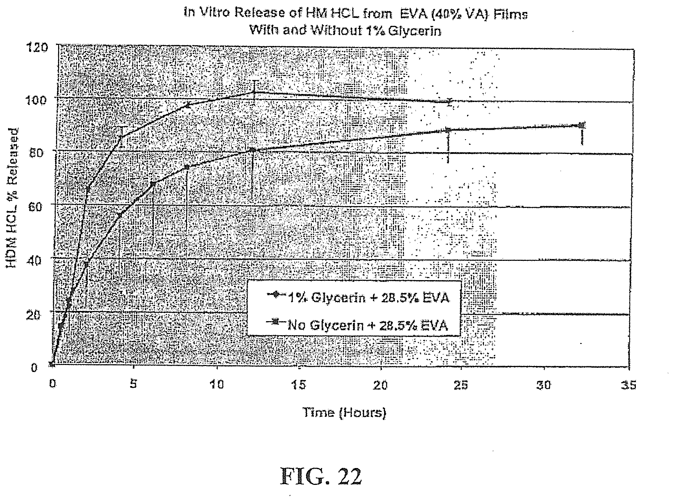

Figure 22 illustrates the results of an *in vitro* dissolution study comparing the percentage of hydromorphone released from a reservoir matrix prepared according to a procedure similar to that of Example 4 and comprising glycerin as a bio-compatible filler, and the percentage of hydromorphone released from a similar reservoir matrix prepared according to a procedure similar to that of Example 6, that does not comprise glycerin as a bio-compatible filler.

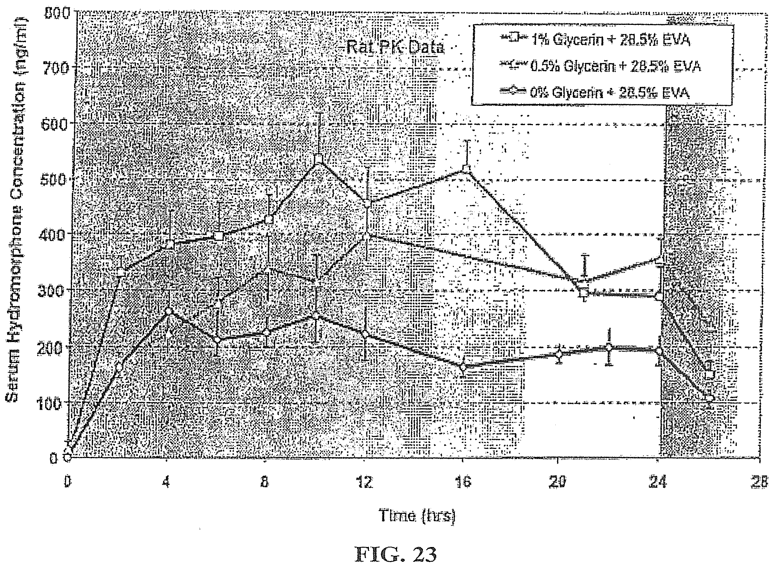

Figure 23 graphically illustrates the results of an *in vivo* hairless rat pharmacokinetic study showing the effect of increasing glycerin levels on steady-state hydromorphone serum levels. In this study, hydromorphone HCL delivery reservoirs prepared according to procedures similar to the exemplary procedures set forth in Examples 4, 5, and 6 above, comprising 1% glycerin, 0.5% glycerin, and 0.0% glycerin, respectively, were each tested on four hairless rats over a 24-hour administration period.

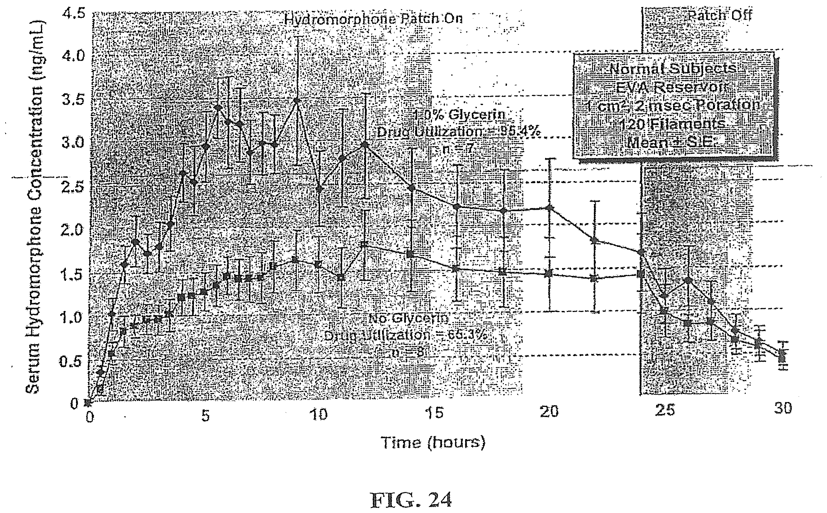

Figure 24 reports the mean serum hydromorphone concentration levels from 7 human test subjects that were administered hydromorphone via a delivery reservoir containing 1.0% glycerin over a 24-hour administration period, as prepared according to a procedure similar to that of Example 4 above. This data is also compared to mean serum hydromorphone concentration levels from 8 human test subjects that were administered hydromorphone via a delivery reservoir containing no glycerin, also over the same 24- hour administration period, and as prepared according to a procedure similar to that of Example 6 above. The permeant reservoirs were tested on human test subjects by applying the reservoirs to a 1 cm² microporated administration site. The administration site was provided on the skin of the test subject by thermal poration using an apparatus having an array of 120 thermal poration filaments, such as the PassPort™ thermal poration system from Altea Therapeutics. The filament array was operated for a 2 millisecond pulse. The resulting PK profiles show that glycerin, similar to the effects of propylene glycol illustrated above, can significantly increase the steady-state serum hydromorphone level achieved as well as the release rate of hydromorphone from the film as evidenced by the increase in drug utilization.

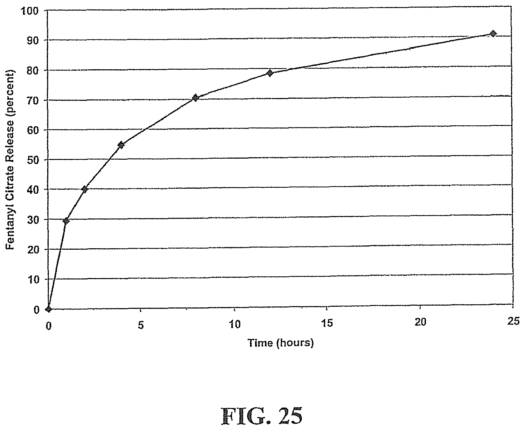

Figure 25 reports the mean pharmacokinetic profile for three lots of an exemplary permeant delivery reservoir device according to the present invention comprising fentanyl citrate as the bio-active agent. Each lot of permeant delivery reservoirs was prepared according to the procedure similar to or the same as that set forth above in Example 7, and comprised approximately 28.5% EVA, 10% fentanyl citrate, and 61.5% mannitol. Four delivery reservoirs from each lot were tested on the abdomen region of hairless rat test subjects by applying the reservoirs to a 1 cm² microporated administration site. The administration site was provided on the skin of the test subject by thermal poration using an apparatus having an array of 120 thermal poration filaments, such as the PassPort™ thermal poration system from Altea Therapeutics. The filament array was operated for a 2 millisecond pulse. The administration period extended for a duration of approximately 24 hours. The resulting mean PK profile indicates the ability for the delivery reservoirs of the present invention to reproducibly provide a relatively steady delivery of fentanyl citrate over a 24-hour administration period.

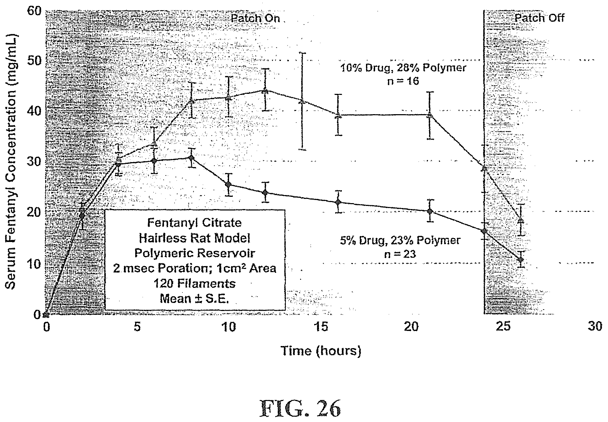

Figure 26 reports by comparison, the mean fentanyl citrate serum level PK profile for permeant delivery reservoirs of the present invention comprising differing concentrations of fentanyl citrate. In particular, shown is a comparison of mean fentanyl citrate serum level PK profiles for delivery reservoirs prepared according to procedures similar to that of Examples 7 and 8, comprising 10% fentanyl citrate and 5% fentanyl citrate, respectively. Figure 26 shows that, in one aspect of the present invention, fentanyl citrate can be delivered through micropores in the skin and that the steady-state level can be controlled by the fentanyl content of the delivery reservoir.

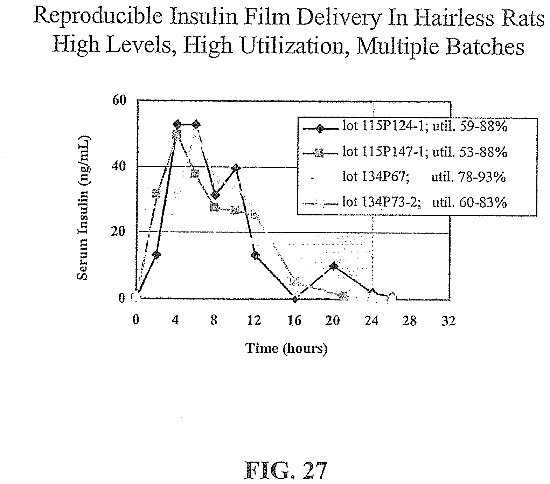

Figure 27 reports the mean insulin serum level PK profiles for four lots of an exemplary permeant delivery reservoir device described herein comprising lyophilized insulin as the bio-active agent. Each lot of permeant delivery reservoirs comprised approximately 20 weight % EVA, approximately 76% excipient, and approximately 4 weight % insulin. The reservoirs were processed via a method of magnetic stirring and solvent casting. Four delivery reservoirs from each lot were tested on the abdomen *region* of *hairless* rat *test* subjects by applying the reservoirs to a 1 cm² microporated administration site. The administration site was provided on the skin of the test subject by thermal poration using an apparatus having an array of 80 thermal poration filaments, such as the PassPort™ thermal poration system from Altea Therapeutics. The filament array was operated for a 7.5 millisecond pulse. Once applied, the administration period extended for a duration of approximately 24 hours. The resulting mean PK profiles for each lot of reservoirs, as shown in Figure 27, indicate the ability for the delivery reservoirs of the present invention to reproducibly provide a relatively steady delivery of insulin over a 24-hour administration period and at a drug utilization rate in the range of, for example, from 53% to 93%.

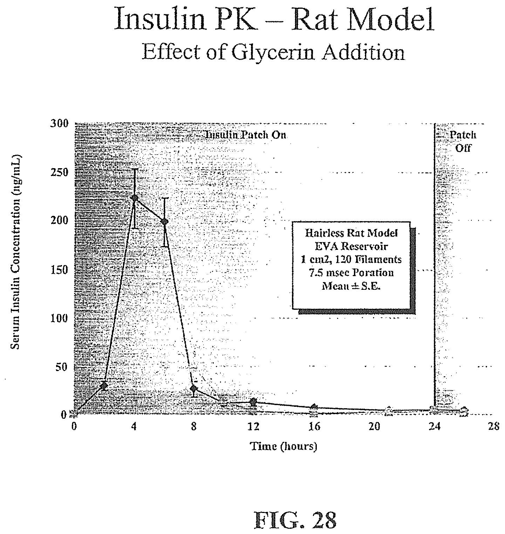

Figure 28 reports, by comparison, the enhancing effect glycerin can have on the peak serum insulin levels in a pharmacokinetic profile study involving hairless rat test subjects. A series of three permeant delivery reservoirs as described in connection with Figure 27 above were again tested on the abdomen region of three hairless rat test subjects. Similarly, a series of three permeant delivery reservoirs comprising approximately 20 weight % EVA, approximately 70.17% excipient, approximately 8 weight % insulin, approximately 1.0 weight % glycerin, and approximately 0.83 weight % cresol, were also tested on the abdomen region of three hairless rat test subjects. Specifically, the subject permeant delivery reservoirs were each applied to a 1 cm² microporated administration site provided on the skin of the test subject by thermal poration using an apparatus having an array of 120 thermal poration filaments, such as the PassPort™ thermal poration system from Altea Therapeutics. The filament array was operated for a 7.5 millisecond pulse. Once applied, the administration period extended for a duration of approximately 24 hours. As shown in Figure 28, the formulation with glycerin resulted in significantly higher mean steady-state serum levels of insulin compared to the formulation without glycerin.

## Claims

1. A device for causing the transdermal flux of a permeant into a subject via at least one formed pathway through a skin layer of the subject, comprising:
an anhydrous delivery reservoir comprising:
i) a non-biodegradable matrix comprising a water-insoluble polymer, having a bottom surface and defining a plurality of conduits therein the matrix, at least a portion of the plurality of conduits being in communication with the bottom surface; and
ii) an undissolved hydrophilic permeant disposed therein at least a portion of the plurality of conduits of the matrix, the hydrophilic permeant comprising at least one bioactive agent and at least one biocompatible filler, wherein the hydrophilic permeant can come in contact with subcutaneous fluid from the subject when the bottom surface of the matrix is positioned in fluid communication with the at least one formed pathway.

2. The device of claim 1, wherein the permeant comprises a plurality of bioactive agents, wherein optionally the device can transdermally deliver at least 10 weight percent; preferably at least 20 weight percent; at least 40 weight percent; at least 60 weight percent; at least about 80 weight percent; or at least 90 weight percent of the permeant disposed in the reservoir matrix.

3. The device of claim 1 or 2, wherein the filler can comprise at least one hygroscopic agent; at least one osmotic agent; mannitol; at least one anti-healing agent; at least one anti-clotting agent; at least one anti-inflammatory agent; at least one reepitheliating inhibitory agent; at least one nitrous oxide inhibitory agent; and/or at least one melanogenesis inhibitory agent, wherein optionally the filler is present in an amount, relative to a predetermined amount of bioactive agent, that can provide a predetermined transdermal dosage of bioactive agent.

4. The device of claim 1, wherein the permeant is present in an amount, relative to a predetermined amount of the solid matrix, that can provide a predetermined rate of transdermal permeant diffusion.

5. The device of claim 1, wherein the delivery reservoir comprises from 20 weight % to 80 weight %, preferably from 20 weight % to 50 weight % matrix.

6. The device of claim 1, wherein the delivery reservoir has a thickness in the range of from 0.01 mm to 10.0 mm, preferably in the range of from 0.5 mm to 1.0 mm.

7. The device of claim 1, wherein the delivery reservoir comprises a plurality of delivery reservoirs in a stacked arrangement, wherein optionally at least two of the plurality of delivery reservoirs comprise a different permeant or wherein optionally each of the plurality of delivery reservoirs comprises a different permeant.

8. The device of claim 7, wherein the plurality of delivery reservoirs can transdermally deliver at least one permeant at a predetermined rate of delivery over a predetermined administration period, wherein optionally the predetermined rate of delivery is variable over the administration period; or wherein optionally the predetermined rate of delivery remains substantially constant over the predetermined administration period, wherein optionally the administration period is from 0.1 hours to 400 hours; from 6 hours to 12 hours; from 12 hours to 30 hours; from 30 hours to 50 hours; from 50 hours to 80 hours.

9. The device of claim 1, wherein the delivery reservoir has a substantially planar and smooth bottom surface; or wherein the delivery reservoir has a textured bottom surface; or wherein the delivery reservoir has a bottom surface and wherein at least a portion of the bottom surface is non-planar.

10. The device of claim 1, further comprising a backing support layer having an inwardly facing surface, wherein the delivery reservoir has a top surface and an opposed bottom surface and wherein at least a portion of the inwardly facing surface of the backing support layer is connected to at least a portion of a top surface of the delivery reservoir.

11. The device of claim 10, further comprising a protective release layer connected to at least a portion of the bottom surface of the composite reservoir, wherein optionally the protective release layer is removably secured to at least a portion of the peripherally extending backing support layer; or wherein optionally when the protective release layer is removed, the bottom surface of the composite reservoir can be positioned in fluid communication with at least one pathway in the skin of a subject.

12. The device of claim 10, wherein a portion of the backing support layer peripherally extends beyond the reservoir and wherein at least a portion of peripherally extending support layer has an adhesive layer deposited on the inward facing surface thereof.

13. The device of claim 1, wherein the delivery reservoir comprises a top surface and an opposed bottom surface and wherein a first electrode is positioned in electrical communication with the top surface and a second electrode is positioned in electrical communication with the bottom surface, optionally further comprising a third electrode remotely positioned from the delivery reservoir and adapted to be positioned in electrical communication with the skin of a subject; or wherein optionally the first and second electrodes are in selective electrical communication with a controllable voltage or current source, wherein optionally the first, second and third electrodes are in selective electrical communication with a controllable voltage or current source.

14. The device of claim 1, **characterized in that** the delivery reservoir has been manufactured by a production method comprising dry-mixing the water-insoluble polymer and the hydrophilic permeant together using a heated kneading mixer, and then processing the resulting heat-kneaded mixture into individual dosage forms of the delivery reservoir; or **characterized in that** the delivery reservoir has been manufactured by a solvent casting process, wherein a matrix material is dissolved into an organic solvent, an undissolved hydrophilic permeant is then added to the dissolved matrix material and the resulting suspension is then poured into reservoir forms having a desired size and shape, and the solvent is then evaporated or otherwise removed to provide the delivery reservoir; or **characterized in that** the delivery reservoir has been manufactured by a production method comprising mixing the hydrophilic permeant and a cryo-milled powder of the water-insoluble polymer until a substantially uniform and homogenous distribution of the hydrophilic permeant and the water-insoluble polymer is achieved, and subjecting the resulting mixture to a hot or cold pressing or melt extrusion process.

15. A system for causing the transdermal flux of a permeant into a subject via at least one formed pathway through a skin layer of the subject, comprising:
a) a means for intentionally forming the at least one formed pathway in the skin layer; and
b) an anhydrous delivery reservoir comprising:
i) a non-biodegradable matrix comprising a water-insoluble polymer, having a bottom surface and defining a plurality of conduits therein the matrix, at least a portion of the plurality of conduits being in communication with the bottom surface; and
ii) an undissolved water-soluble permeant disposed therein at least a portion of the plurality of conduits of the matrix, the water-soluble permeant comprising at least one bioactive agent and at least one biocompatible filler, wherein the water-soluble permeant can come in contact with subcutaneous fluid when the bottom surface of the matrix is positioned in fluid communication with the at least one formed pathway.

## Patentansprüche

1. Vorrichtung zum Veranlassen des transdermalen Flusses eines Permeationsmittels in ein Wesen über mindestens einen geformten Weg durch eine Hautschicht des Wesens, die
einen wasserfreien Abgabebehälter aufweist, der
i) eine nicht biologisch abbaubare Matrix, die ein wasserunlösliches Polymer aufweist, die eine untere Oberfläche hat und eine Vielzahl von Leitungen in der Matrix definiert, wobei mindestens ein Teil der Vielzahl von Leitungen mit der unteren Oberfläche in Verbindung steht; und
ii) ein ungelöstes hydrophiles Permeationsmittel aufweist, das in mindestens einem Teil der Vielzahl von Leitungen der Matrix angeordnet ist, wobei das hydrophile Permeationsmittel mindestens ein bioaktives Mittel und mindestens einen biokompatiblen Füllstoff aufweist, wobei das hydrophile Permeationsmittel mit subkutaner Flüssigkeit von dem Wesen in Berührung kommen kann, wenn die untere Oberfläche der Matrix in Fluidverbindung mit dem mindestens einen geformten Weg steht.

2. Vorrichtung nach Anspruch 1, wobei das Permeationsmittel eine Vielzahl von bioaktiven Mitteln aufweist, wobei gegebenenfalls die Vorrichtung mindestens 10 Gewichtsprozent; vorzugsweise mindestens 20 Gewichtsprozent; mindestens 40 Gewichtsprozent; mindestens 60 Gewichtsprozent; mindestens etwa 80 Gewichtsprozent; oder mindestens 90 Gewichtsprozent des in der Behältermatrix angeordneten Permeationsmittels transdermal abgeben kann.

3. Vorrichtung nach Anspruch 1 oder 2, wobei der Füllstoff mindestens ein hygroskopisches Mittel; mindestens ein osmotisches Mittel; Mannit; mindestens ein Anti-Heilmittel; mindestens ein Anti-Gerinnungsmittel; mindestens ein entzündungshemmendes Mittel; mindestens ein die Re-Epithelisierung hemmendes Mittel; mindestens ein Stickoxid hemmendes Mittel; und/oder mindestens ein die Melanogenese hemmendes Mittel aufweisen kann, wobei gegebenenfalls der Füllstoff in einer Menge, bezogen auf eine vorbestimmte Menge an bioaktivem Mittel, vorhanden ist, die eine vorbestimmte transdermale Dosierung von bioaktivem Mittel bereitstellen kann.

4. Vorrichtung nach Anspruch 1, wobei das Permeationsmittel in einer Menge, bezogen auf eine vorbestimmte Menge der festen Matrix, vorhanden ist, die eine vorbestimmte Rate der transdermalen Permeationsmittel-Diffusion bereitstellen kann.

5. Vorrichtung nach Anspruch 1, wobei der Abgabebehälter 20 Gew.-% bis 80 Gew.-%, vorzugsweise 20 Gew.-% bis 50 Gew.-% Matrix aufweist.

6. Vorrichtung nach Anspruch 1, wobei der Abgabebehälter eine Dicke im Bereich von 0,01 mm bis 10,0 mm, vorzugsweise im Bereich von 0,5 mm bis 1,0 mm hat.

7. Vorrichtung nach Anspruch 1, wobei der Abgabebehälter eine Vielzahl von Abgabebehältern in einer gestapelten Anordnung aufweist, wobei gegebenenfalls mindestens zwei der Vielzahl von Abgabebehältern ein unterschiedliches Permeationsmittel aufweisen, oder wobei gegebenenfalls jeder der Vielzahl von Abgabebehältern ein unterschiedliches Permeationsmittel aufweist.

8. Vorrichtung nach Anspruch 7, wobei die Vielzahl von Abgabebehältern mindestens ein Permeationsmittel mit einer vorgegebenen Abgaberate über eine vorgegebene Verabreichungsdauer transdermal abgeben kann, wobei gegebenenfalls die vorgegebene Abgaberate über die Verabreichungsdauer variabel ist; oder wobei gegebenenfalls die vorgegebene Abgaberate über die vorgegebene Verabreichungsdauer im Wesentlichen konstant bleibt, wobei gegebenenfalls die Verabreichungsdauer 0,1 Stunden bis 400 Stunden; 6 Stunden bis 12 Stunden; 12 Stunden bis 30 Stunden; 30 Stunden bis 50 Stunden; oder 50 Stunden bis 80 Stunden beträgt.

9. Vorrichtung nach Anspruch 1, wobei der Abgabebehälter eine im Wesentlichen ebene und glatte untere Oberfläche hat; oder wobei der Abgabebehälter eine strukturierte untere Oberfläche hat; oder wobei der Abgabebehälter eine untere Oberfläche hat und wobei mindestens ein Teil der unteren Oberfläche nicht eben ist.

10. Vorrichtung nach Anspruch 1, die ferner eine stützende Trägerschicht aufweist, die eine nach innen gerichtete Oberfläche hat, wobei der Abgabebehälter eine obere Oberfläche und eine gegenüberliegende untere Oberfläche hat und wobei mindestens ein Teil der nach innen gerichteten Oberfläche der stützenden Trägerschicht mit mindestens einem Teil einer oberen Oberfläche des Abgabebehälters verbunden ist.

11. Vorrichtung nach Anspruch 10, die ferner eine schützende Trennschicht aufweist, die mit mindestens einem Teil der unteren Oberfläche des Verbundbehälters verbunden ist, wobei gegebenenfalls die schützende Trennschicht lösbar an mindestens einem Teil der sich peripher erstreckenden stützenden Trägerschicht befestigt ist; oder wobei gegebenenfalls die untere Oberfläche des Verbundbehälters in Fluidverbindung mit mindestens einem Weg in der Haut eines Wesens stehen kann, wenn die schützende Trennschicht entfernt ist.

12. Vorrichtung nach Anspruch 10, wobei sich ein Teil der stützenden Trägerschicht peripher über den Behälter hinaus erstreckt und wobei mindestens ein Teil der sich peripher erstreckenden Trägerschicht eine Klebeschicht aufweist, die auf der nach innen gerichteten Oberfläche davon aufgebracht ist.

13. Vorrichtung nach Anspruch 1, wobei der Abgabebehälter eine obere Oberfläche und eine gegenüberliegende untere Oberfläche aufweist, und wobei eine erste Elektrode in elektrischer Verbindung mit der oberen Oberfläche steht, und eine zweite Elektrode in elektrischer Verbindung mit der unteren Oberfläche steht, wobei gegebenenfalls der Abgabebehälter ferner eine dritte Elektrode aufweist, die entfernt von dem Abgabebehälter positioniert ist, und angepasst ist, um in elektrischer Verbindung mit der Haut eines Wesens zu stehen; oder wobei gegebenenfalls die erste und die zweite Elektrode in selektiver elektrischer Verbindung mit einer steuerbaren Spannungs- oder Stromquelle stehen, wobei gegebenenfalls die erste, zweite und dritte Elektrode in selektiver elektrischer Verbindung mit einer steuerbaren Spannungs- oder Stromquelle stehen.

14. Vorrichtung nach Anspruch 1, **dadurch gekennzeichnet, dass** der Abgabebehälter durch ein Herstellungsverfahren hergestellt wurde, bei dem das wasserunlösliche Polymer und das hydrophile Permeationsmittel unter Verwendung eines beheizten Mischkneters trocken vermischt werden, und dann die resultierende wärmeverknetete Mischung in einzelne Dosierungsformen des Abgabebehälters verarbeitet wird; oder **dadurch gekennzeichnet, dass** der Abgabebehälter durch ein Lösungsmittelgussverfahren hergestellt wurde, wobei ein Matrixmaterial in einem organischen Lösungsmittel gelöst wird, dann dem gelösten Matrixmaterial ein ungelöstes hydrophiles Permeationsmittel zugesetzt wird, und die resultierende Suspension dann in Behälterformen mit einer gewünschten Größe und Form gegossen wird, und das Lösungsmittel dann verdampft oder auf andere Weise entfernt wird, um den Abgabebehälter zu liefern; oder **dadurch gekennzeichnet, dass** der Abgabebehälter durch ein Herstellungsverfahren hergestellt wurde, wobei das hydrophile Permeationsmittel und ein kryo-gemahlenes Pulver des wasserunlöslichen Polymers gemischt werden, bis eine im Wesentlichen gleichmäßige und homogene Verteilung des hydrophilen Permeationsmittels und des wasserunlöslichen Polymers erreicht ist, und die erhaltene Mischung einem Heiß- oder Kaltpress- oder Schmelzextrusionsverfahren unterworfen wird.

15. System zum Veranlassen des transdermalen Flusses eines Permeationsmittels in ein Wesen über mindestens einen geformten Weg durch eine Hautschicht des Wesens, das
a. ein Mittel zum absichtlichen Bilden des mindestens einen geformten Wegs in der Hautschicht; und
b. einen wasserfreien Abgabebehälter aufweist, der
i) eine nicht biologisch abbaubare Matrix, die ein wasserunlösliches Polymer aufweist, die eine untere Oberfläche hat und eine Vielzahl von Leitungen in der Matrix definiert, wobei mindestens ein Teil der Vielzahl von Leitungen mit der unteren Oberfläche in Verbindung steht; und
ii) ein ungelöstes hydrophiles Permeationsmittel aufweist, das in mindestens einem Teil der Vielzahl von Leitungen der Matrix angeordnet ist, wobei das hydrophile Permeationsmittel mindestens ein bioaktives Mittel und mindestens einen biokompatiblen Füllstoff aufweist, wobei das hydrophile Permeationsmittel mit subkutaner Flüssigkeit von dem Wesen in Berührung kommen kann, wenn die untere Oberfläche der Matrix in Fluidverbindung mit dem mindestens einen geformten Weg steht.

## Revendications

1. Dispositif pour générer le flux transdermique d'un perméant chez un sujet via au moins une voie formée à travers une couche de peau du sujet, comprenant :
un réservoir de délivrance anhydre comprenant :
i) une matrice non biodégradable comprenant un polymère insoluble dans l'eau, possédant une surface de fond et définissant une pluralité de conduites à l'intérieur de la matrice, au moins une partie de la pluralité de conduites étant en communication avec la surface de fond ; et
ii) un perméant hydrophile non dissous disposé à l'intérieur d'au moins une partie de la pluralité de conduites de la matrice, le perméant hydrophile comprenant au moins un agent bioactif et au moins une charge biocompatible, dans lequel le perméant hydrophile peut entrer en contact avec du fluide sous-cutané du sujet lorsque la surface de fond de la matrice est positionnée en communication fluidique avec l'au moins une voie formée.

2. Dispositif selon la revendication 1, dans lequel le perméant comprend une pluralité d'agents bioactifs, dans lequel facultativement le dispositif peut délivrer par voie transdermique au moins 10 pour cent en poids ; de préférence au moins 20 pour cent en poids ; au moins 40 pour cent en poids au moins 60 pour cent en poids ; au moins environ 80 pour cent en poids ; ou au moins 90 pour cent en poids du perméant disposé dans la matrice de réservoir.

3. Dispositif selon la revendication 1 ou 2, dans lequel la charge peut comprendre au moins un agent hygroscopique ; au moins un agent osmotique ; le mannitol ; au moins un agent anticicatrisant ; au moins un agent anticoagulant ; au moins un agent anti-inflammatoire ; au moins un agent inhibiteur de la réépithélialisation ; au moins un agent inhibiteur de l'oxyde nitreux ; et/ou au moins un agent inhibiteur de la mélanogenèse, dans lequel facultativement la charge est présente en une quantité, par rapport à une quantité prédéterminée d'agent bioactif, qui peut fournir un dosage transdermique prédéterminé d'agent bioactif.

4. Dispositif selon la revendication 1, dans lequel le perméant est présent en une quantité, par rapport à une quantité prédéterminée de la matrice solide, qui peut fournir un rythme prédéterminé de diffusion de perméant transdermique.

5. Dispositif selon la revendication 1, dans lequel le réservoir de délivrance comprend de 20 % en poids à 80 % en poids, de préférence de 20 % en poids à 50 % en poids, de matrice.

6. Dispositif selon la revendication 1, dans lequel le réservoir de délivrance possède une épaisseur dans la plage de 0,01 mm à 10,0 mm, de préférence dans la plage de 0,5 mm à 1,0 mm.

7. Dispositif selon la revendication 1, dans lequel le réservoir de délivrance comprend une pluralité de réservoirs de délivrance dans un agencement empilé, dans lequel facultativement au moins deux de la pluralité de réservoirs de délivrance comprennent un perméant différent ou dans lequel facultativement chacun de la pluralité de réservoirs de délivrance comprend un perméant différent.

8. Dispositif selon la revendication 7, dans lequel la pluralité de réservoirs de délivrance peuvent délivrer par voie transdermique au moins un perméant à un rythme prédéterminé de délivrance pendant une période d'administration prédéterminée, dans lequel facultativement le rythme prédéterminé de délivrance est variable pendant la période d'administration ; ou dans lequel facultativement le rythme prédéterminé de délivrance reste sensiblement constant pendant la période d'administration prédéterminée, dans lequel facultativement la période d'administration est de 0,1 heure à 400 heures ; de 6 heures à 12 heures ; de 12 heures à 30 heures ; de 30 heures à 50 heures ; de 50 heures à 80 heures.

9. Dispositif selon la revendication 1, dans lequel le réservoir de délivrance possède une surface de fond sensiblement planaire et lisse ; ou dans lequel le réservoir de délivrance possède une surface de fond texturée ; ou dans lequel le réservoir de délivrance possède une surface de fond et dans lequel au moins une partie de la surface de fond est non planaire.

10. Dispositif selon la revendication 1, comprenant en outre une couche de support de base possédant une surface orientée vers l'intérieur, dans lequel le réservoir de délivrance possède une surface supérieure et une surface de fond opposée et dans lequel au moins une partie de la surface orientée vers l'intérieur de la couche de support de base est reliée à au moins une partie d'une surface supérieure du réservoir de délivrance.

11. Dispositif selon la revendication 10, comprenant en outre une couche de séparation protectrice reliée à au moins une partie de la surface de fond du réservoir composite, dans lequel facultativement la couche de séparation protectrice est fixée de manière amovible à au moins une partie de la couche de support de base s'étendant de manière périphérique ; ou dans lequel facultativement lorsque la couche de séparation protectrice est enlevée, la surface de fond du réservoir composite peut être positionnée en communication fluidique avec au moins une voie dans la peau d'un sujet.

12. Dispositif selon la revendication 10, dans lequel une partie de la couche de support de base s'étend de manière périphérique au-delà du réservoir et dans lequel au moins une partie de la couche de support s'étendant de manière périphérique possède une couche adhésive déposée sur la surface de celle-ci orientée vers l'intérieur.

13. Dispositif selon la revendication 1, dans lequel le réservoir de délivrance comprend une surface supérieure et une surface de fond opposée et dans lequel une première électrode est positionnée en communication électrique avec la surface supérieure et une deuxième électrode est positionnée en communication électrique avec la surface de fond, facultativement comprenant en outre une troisième électrode positionnée à distance du réservoir de délivrance et adaptée pour être positionnée en communication électrique avec la peau d'un sujet ; ou dans lequel facultativement les première et deuxième électrodes sont en communication électrique sélective avec une source de tension ou de courant réglable, dans lequel facultativement les première, deuxième et troisième électrodes sont en communication électrique sélective avec une source de tension ou de courant réglable.

14. Dispositif selon la revendication 1, **caractérisé en ce que** le réservoir de délivrance a été fabriqué par un procédé de production comprenant le mélange à sec du polymère insoluble dans l'eau et du perméant hydrophile ensemble en utilisant un mélangeur malaxeur chauffé, et ensuite le traitement du mélange malaxé à chaud résultant en formes de dosage individuelles du réservoir de délivrance ; ou **caractérisé en ce que** le réservoir de délivrance a été fabriqué par un processus de coulée avec un solvant, dans lequel un matériau de matrice est dissous dans un solvant organique, un perméant hydrophile non dissous est ensuite ajouté au matériau de matrice dissous et la suspension résultante est ensuite versée dans des formes de réservoir possédant une taille et une configuration souhaitées, et le solvant est ensuite évaporé ou autrement enlevé pour fournir le réservoir de délivrance ; ou **caractérisé en ce que** le réservoir de délivrance a été fabriqué par un procédé de production comprenant le mélange du perméant hydrophile et d'une poudre pulvérisée à l'état congelé du polymère insoluble dans l'eau jusqu'à ce qu'une répartition sensiblement uniforme et homogène du perméant hydrophile et du polymère insoluble dans l'eau soit obtenue, et la soumission du mélange résultant à un processus de pressage à chaud ou à froid ou d'extrusion à l'état fondu.

15. Système pour générer le flux transdermique d'un perméant chez un sujet via au moins une voie formée à travers une couche de peau du sujet, comprenant :
a) un moyen pour former intentionnellement l'au moins une voie formée dans la couche de peau ; et
b) un réservoir de délivrance anhydre comprenant :
i) une matrice non biodégradable comprenant un polymère insoluble dans l'eau, possédant une surface de fond et définissant une pluralité de conduites à l'intérieur de la matrice, au moins une partie de la pluralité de conduites étant en communication avec la surface de fond ; et
ii) un perméant soluble dans l'eau non dissous disposé à l'intérieur d'au moins une partie de la pluralité de conduites de la matrice, le perméant soluble dans l'eau comprenant au moins un agent bioactif et au moins une charge biocompatible, dans lequel le perméant soluble dans l'eau peut entrer en contact avec du fluide sous-cutané lorsque la surface de fond de la matrice est positionnée en communication fluidique avec l'au moins une voie formée.
